(19) Europäisches Patentamt
European Patent Office
Office européen des brevets

(11) **EP 2 875 828 B1**

(12) **EUROPEAN PATENT SPECIFICATION**

(45) Date of publication and mention
of the grant of the patent:
**01.05.2019 Bulletin 2019/18**

(21) Application number: **13766388.6**

(22) Date of filing: **19.07.2013**

(51) Int Cl.:
**A61K 39/35** (2006.01)    **A61K 39/36** (2006.01)
**A61P 37/08** (2006.01)

(86) International application number:
**PCT/ES2013/070527**

(87) International publication number:
**WO 2014/013123 (23.01.2014 Gazette 2014/04)**

(54) **COMPOSITION FOR INTRADERMAL ADMINISTRATION OF POLYMERIZED ALLERGENS**

COMPOSICIÓN PARA LA ADMINISTRACIÓN INTRADÉRMICA DE ALERGOIDES

COMPOSITION POUR L'ADMINISTRATION INTRADERMIQUE D'ALLERGOÏDES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **20.07.2012 ES 201231168**
**08.03.2013 ES 201330335**

(43) Date of publication of application:
**27.05.2015 Bulletin 2015/22**

(73) Proprietor: **Diater, Laboratorio de Diagnóstico y Aplicaciones Terapéuticas, S.A.**
**28918 Leganés, Madrid (ES)**

(72) Inventors:
• **PALACIOS PELÁEZ, Ricardo**
28918 Leganés Madrid (ES)
• **RODRÍGUEZ GIL, David**
28918 Leganés Madrid (ES)
• **ALCOVER DÍAZ, Javier**
28918 Leganés Madrid (ES)
• **PINEDA DE LA LOSA, Fernando**
28918 Leganés Madrid (ES)

(74) Representative: **ABG Intellectual Property, S.L.**
**Avenida de Burgos, 16D**
**Edificio Euromor**
**28036 Madrid (ES)**

(56) References cited:
**EP-A1- 0 782 860        WO-A1-2011/026641**
**WO-A1-2011/098569        GB-A- 1 282 163**
**US-A- 4 180 562        US-A- 4 269 764**
**US-A- 4 473 495**

• **MALET A ET AL: "Clinical and immunological effects of immunotherapy with glutaraldehyde modified house dust mite extract", ALLERGOLOGIA ET IMMUNOPATHOLOGIA, GARSI, MADRID, ES, vol. 22, no. 5, 1 September 1994 (1994-09-01), pages 226-232, XP008098988, ISSN: 0301-0546**
• **ARTS JOSJE H E ET AL: "Dose-response relationships and threshold levels in skin and respiratory allergy", CRITICAL REVIEWS IN TOXICOLOGY, CRC PRESS, BOCA RATON, FL, US, vol. 36, no. 3, 1 March 2006 (2006-03-01), pages 219-251, XP008166098, ISSN: 1040-8444, DOI: 10.1080/10408440500534149**

**Description**

Field of the Invention

The invention relates to a pharmaceutical composition comprising at least one allergoid obtained from an allergen extract or from an individual allergen, for intradermal administration of said allergoid for use in the treatment of allergies.

Background of the Invention

In general terms, an allergy is an abnormal or exaggerated reaction or response of the immune system to substances known as allergens that leads to the synthesis of IgE class (IgE) antibodies or immunoglobulins in allergic individuals, both humans and in some animal species (cats, dogs, horses, etc.), and that are, however, perfectly tolerated in the healthy population.

Allergens can be found in foods, plant pollen, dust mites, animal dander, insect venoms, etc. The IUIS (International Union of Immunological Societies) has established a nomenclature system for identifying allergens present in a source of sensitization consisting of using the first three letters of the genus of the source of sensitization, followed by the first letter of the species and by an Arabic number indicating the order of discovery of the allergen. By way of illustration, in the case of the domestic dust mite *Dermatophagoides pteronyssinus,* its first discovered allergen was called Derp 1.

The pathophysiology of the IgE-mediated allergic reaction (Type I) is clearly seen in allergic patients, both in human patients and in some animal species, after the allergen binds to the allergen-specific IgEs fixed on the surface of mast cells or mastocytes. The signals produced after the binding lead to the release of the mediators stored in the mast cell. The mediators can be pre-formed (histamine, tryptase, etc.) or they can correspond to subsequent synthesis by cyclooxygenase (prostaglandin D2 and thromboxane A2) and lipoxygenase (leukotrienes B4, C4, D4, E4), as well as to cytokine secretion. Histamine is the mediator responsible for most of the phenomena associated with the immediate phase allergic reaction.

Etiological diagnosis (source of sensitization) in allergic patients is usually done by means of the so-called skin tests, which can be classified as prick tests or intradermal skin tests, with the allergen extract obtained from the source of sensitization. When the allergen extract is introduced into the skin, a local inflammatory reaction occurs in the form of a papule and erythema a few minutes later which gives rise to what is known as an immediate response. The measurement of the areas or diameters of the responses in the form of a papule or erythema allows establishing the etiological diagnosis as well as the patient sensitivity to the source of sensitization or allergen extract used. The intradermal skin test is widely documented as being more sensitive than the prick test, and it generally uses dilutions between 1:100 and 1:1,000 of the concentrations used in prick tests. Another alternative for etiological diagnosis which shows an elevated correlation with the skin test is the quantitation of allergen extract-specific IgE present in patient serum by means of ELISA techniques.

The treatment of the allergic diseases initially starts by establishing the suitable etiological diagnosis that allows identifying the source of allergic sensitization and includes a series of preventive and therapeutic measures. Preventing or minimizing exposure to the source of allergic sensitization is the first step of treatment. The second step includes pharmacological treatment of allergic patients and comprises (i) preventive treatment that leads to preventing or minimizing the mast cell mediator release which, regardless of the pathology, can use various medicinal products, such as chromones or theophyllines, (ii) symptomatic treatment to prevent mediator-induced biological effects once said mediators have been released, for which antihistamines or antileukotrienes are used, and (iii) a background treatment which allows solving the inflammatory phenomena occurring in the target organ (rhinitis, asthma), for which, for example, corticosteroids are used.

Etiological (specific) treatment of the allergy includes immunotherapy. Immunotherapy with allergen extracts obtained from the source of sensitization is an alternative treatment that is concomitant with pharmacological treatment, recognized by the WHO (World Health Organization) as the only treatment capable of returning the impaired immune response back to normal in an allergic patient. In general terms, it comprises the administration of increasing doses (volume and concentration) of the allergen extract until reaching a maximum maintenance dose. Conventional specific allergen extract immunotherapy has been exclusively administering the extract by subcutaneous route for more than 100 years, and recently another new administration route, i.e., sublingual route, has come about, and in terms of dosing regimen, volume and concentrations that are 10 times greater than those used in the conventional cutaneous administration route, the sublingual administration route can be an alternative. Document GB1282163A discloses the subcutaneous administration of an allergoid-containing composition for treating an allergy to an allergen in a subject that suffers from said allergy and that dosage rates vary according to the allergic condition of the patient. Document US4269764A discloses the use of an allergoid-containing composition that is intracutaneously administered at a volume of 0.02 mL within the context of a cutaneous titration. Malet et al., Allergologia et Immunopathologia, Garsi, Madrid, ES, vol. 22, no. 5, 1 September 1994 disclose the subcutaneous administration of an allergoid-containing composition for treating an allergy to house dust mite.

The problem resulting from the clinical practice of specific allergen extract immunotherapy by either the subcutaneous or sublingual route is common and relates to safety in terms of their ability to induce allergic responses after the administration of the doses, and particularly in escalating to the maintenance dose. This means that the patient must be kept under observation for at least 20 minutes after administration of each dose. On the other hand, the dosing regimen until reaching the maximum dose involves tedious dose-finding tests according to patient sensitivity, in addition to generating many types of dosage regimens to minimize the number of doses to be administered until reaching the maintenance dose.

Therefore, there is a need to develop an alternative system to those already existing which allows overcoming all or some of the problems mentioned above; specifically, it would be desirable to increase safety of immunotherapy treatment in terms of reducing allergic responses after administering the doses and in escalating to the maintenance dose; it would also be desirable to reduce tedious dose-finding tests until reaching the maximum dose to be administered to patients and to minimize the number of doses to be administered until reaching the maintenance dose.

Brief Description of the Invention

The inventors have found that the so-called allergoids obtained by means of polymerizing allergen extracts of, for example, *Phleum pratense* (Examples 1-5), *Parietaria judaica* and *Artemisia vulgaris* (Example 7), *Dermatophagoides pteronyssinus* (Examples 8 and 9) and *Apis mellifera* (Examples 10 and 11), with glutaraldehyde, show lower allergenicity (ability to bind to allergen-specific IgE) than the corresponding native allergen extracts, as clearly shown in the histamine release and $Ag_{50}$ value determinations, but they maintain the immunogenicity, as shown by the results derived from the immunization studies conducted with native *Phleum pratense* allergen extracts and glutaraldehyde-polymerized *Phleum pratense* allergen extracts in rabbits, using the subcutaneous and intradermal administration routes.

The invention contemplates allergoids to be administered by intradermal route for use in treating allergic diseases by means of immunotherapy. An advantage associated with it consists of eliminating the escalation dose to reach the maximum dose tolerated by the patient, given that in the design of the allergoid (chemically modified allergen extract, for example, a glutaraldehyde-polymerized allergen extract), a single administration dose established in terms of safety is considered as the allergoid dilution which makes the intradermal skin test performed with it negative. The invention shows that this dose is safe given that diagnosis and immunotherapy are performed through the same intradermal route, and it is therefore possible to establish for each allergen the maximum tolerated dose depending on patient sensitivity since it is tested on a population that is specifically sensitized to the allergen in question. Furthermore, the invention eliminates the diversity of the volumes to be administered in allergen extract immunotherapy, which is a factor that causes the possibility of inducing overdose reactions in clinical practice. In fact, the maximum volume to be administered will always be 0.1 mL, given that this limit is inherent to the intradermal administration technique.

Therefore, in one aspect the invention relates to a pharmaceutical composition for use in the treatment of a subject suffering from an allergy to an allergen, wherein the composition is to be administered intradermally, the composition comprising at least one allergoid and at least one excipient, wherein said allergoid is an allergoid obtained by means of chemically modifying said allergen, wherein said allergoid is present in the composition at the non-reactive maximum dose and wherein said non-reactive maximum dose corresponds to the allergoid concentration that, administered by the intradermal route, makes the skin test negative as tested on a population that is specifically sensitized to said allergen by means of an end-point titration study with the allergoid in a population of patients sensitized to the allergen.

In another aspect, the invention relates to a pharmaceutical kit comprising a pharmaceutical composition wherein the composition is to be administered intradermally, the composition comprising at least one allergoid and at least one excipient, wherein said allergoid is an allergoid obtained by means of chemically modifying said allergen, wherein said allergoid is present in the composition at the non-reactive maximum dose and wherein said non-reactive maximum dose corresponds to the allergoid concentration that, administered by the intradermal route, makes the skin test negative as tested on a population that is specifically sensitized to said allergen by means of an end-point titration study with the allergoid in a population of patients sensitized to the allergen and the means and instructions for administering said composition for use in the treatment of a subject suffering from an allergy to an allergen.

Brief Description of the Drawings

Figure 1 shows an anti-serum titer obtained in rabbit immunized with native *Phleum pratense* administered subcutaneously.

Figure 2 shows an anti-serum titer obtained in rabbit immunized with glutaraldehyde-polymerized *Phleum pratense* administered subcutaneously.

Figure 3 shows an anti-serum titer obtained in rabbit immunized with native *Phleum pratense* administered intradermally.

Figure 4 shows an anti-serum titer obtained in rabbit immunized with glutaraldehyde-polymerized *Phleum pratense* administered intradermally.

Figure 5 shows the histamine release of a native *Phleum pratense* allergen extract compared to a glutaraldehyde-polymerized *Phleum pratense* allergen extract using human whole blood.

Figure 6 shows a comparison between native *Parietaria judaica* extract (Par j 001-10, lane 2) and glutaraldehyde-polymerized *Parietaria judaica* extract (Par j 003p-11, lanes 3, 4 and 5) determined by means of polyacrylamide gel electrophoresis and Coomassie blue staining.

Figure 7 shows a comparison between native *Parietaria judaica* extract (Par j 001-10, lane 2) and glutaraldehyde-polymerized *Parietaria judaica* extract (Par j 003p-11, lane 3) determined by means of immunoblotting.

Figure 8 shows a comparison between native *Parietaria judaica* extract (Par j 001-10) and glutaraldehyde-polymerized *Parietaria judaica* extract (Par j 003p-11) determined by means of ELISA inhibition, showing a loss of potency of 91.46%.

Figure 9 shows a comparison between native *Artemisia vulgaris* extract (Art v 001-08 and Art v 001-10, lanes 2 and 3) and glutaraldehyde-polymerized *Artemisia vulgaris* extract (Art v 002p-12, lanes 4, 5 and 6) determined by means of polyacrylamide gel electrophoresis and Coomassie blue staining.

Figure 10 shows a comparison between native *Artemisia vulgaris* extract (Art v 001-06 and Art v 001-10, lanes 2 and 3) and glutaraldehyde-polymerized *Artemisia vulgaris* extract (Art v 002p-12, lane 4) determined by means of immunoblotting.

Figure 11 shows a comparison between native *Artemisia vulgaris* extract (Art v 001-10) and glutaraldehyde-polymerized *Artemisia vulgaris* extract (Art v 002p-12) determined by means of ELISA inhibition, showing a loss of potency of 98.91%.

Figure 12 shows a comparison between native *Dermatophagoides pteronyssinus* extract (Der p SAP 001-13, lane 1) and glutaraldehyde-polymerized *Dermatophagoides pteronyssinus* extract (lyophilized polymerized Der p, lane 2) determined by means of polyacrylamide gel electrophoresis and Coomassie blue staining.

Figure 13 shows a comparison between native *Dermatophagoides pteronyssinus* extract (Der p 001-13, lane 1) and glutaraldehyde-polymerized *Dermatophagoides pteronyssinus* extract (lyophilized polymerized Der p, lane 2) determined by means of immunoblotting.

Figure 14 shows a comparison between native *Dermatophagoides pteronyssinus* extract, (Der p 001-13) and 3 consecutive batches of lyophilized and glutaraldehyde-polymerized *Dermatophagoides pteronyssinus* allergen extract determined by means of ELISA inhibition, showing a loss of potency of over 99% in the 3 cases.

Figure 15 shows a comparison between *Apis mellifera* venom raw material (lane 1) and glutaraldehyde-polymerized *Apis mellifera* venom allergen extract (lane 2) determined by means of polyacrylamide gel electrophoresis and Coomassie blue staining.

Figure 16 shows a comparison between *Apis mellifera* venom raw material (lane 1) and glutaraldehyde-polymerized *Apis mellifera* venom allergen extract (lane 2) determined by means of immunoblotting.

Figure 17 shows a comparison between *Apis mellifera* venom raw material and lyophilized glutaraldehyde-polymerized *Apis mellifera* venom allergen extract determined by means of ELISA inhibition, showing a loss of potency of over 99%.

Figure 18 shows the histamine release of a native *Apis mellifera* venom allergen extract compared to a glutaraldehyde-polymerized *Apis mellifera* venom allergen extract using human whole blood.

Detailed Description of the Invention

Composition

It is disclosed a pharmaceutical composition, hereinafter "composition ", for intradermal administration comprising at least one allergoid and at least one excipient.

As it is used herein, a pharmaceutical composition is a composition comprising at least one biologically active (bioactive) agent that is suitable for being administered to a human or another animal and is useful for maintaining a health condition, controlling, alleviating or treating the symptoms, conditions or diseases of an allergic etiology.

Intradermal administration is one of the four types of parenteral administration of bioactive agents. Parenteral administration is a type of administration that introduces the bioactive agent directly into the organism, and therefore provides said bioactive agent directly in systemic circulation. The other 3 types of parenteral administration are the subcutaneous, intramuscular and intravenous routes. Each type of parenteral administration has its own particularities [GUIA PARA LA ADMINISTRACIÓN SEGURA DE MEDICAMENTOS VIA PARENTERAL. May 2011. Coordinator: Ernesto Sanchez Gómez. Pub. "Juan Ramón Jiménez" Hospital. Huelva. ISBN: 978-84-694-1318-0].

According to the present invention, "intradermal administration" is understood as administration in which a pharmaceutical composition comprising a reduced amount of a bioactive agent is injected by means of a fine-gauge needle such that an intracutaneous or intradermal papule is formed. The administration areas generally include the anterior or ventral surface of both forearms, the anterior and superior part of the chest below the clavicles (except in women), and the superior part of the back in the inferior scapular area. This administration route only allows small volumes, normally between 0.05 and 0.1 mL. Given that the dermis is not very irrigated, the effect of the bioactive agent is prolonged for a fair amount of time. This is the route normally used for intradermal reaction diagnostic skin tests in allergies. Needles with a length of 9.5-16 mm, a gauge of 25-26 G (0.5 mm) and a short bezel are generally used for intradermal administration.

As it is used herein, the expression "for intradermal administration" is understood to mean the same as appropriate or suitable for intradermal administration; i.e., in other words, the disclosed composition is formed and processed such that it is suitable for intradermal administration according to generally accepted criteria.

An "excipient" is any pharmacologically inert, pharmaceutically acceptable substance or mixture of substances useful for formulating a pharmaceutical composition. For use in the present invention, the excipient or excipients must be excipient(s) useful for producing pharmaceutical compositions for intradermal administration.

Examples of potentially useful excipients include solvents, co-solvents, diluents, surfactants, co-surfactants, thickeners, stabilizers, antioxidants, solubilizers, pH adjusting agents, dyes and the like. In a particular disclosure, the composition contains a diluent, physiological saline solution (0.9% NaCl) and, optionally, one or more additional pharmaceutically acceptable excipients. In another particular disclosure, said excipient comprises mannitol, for example, mannitol at 10 mg/mL.

Therefore, the disclosed composition is an "intradermal pharmaceutical composition", or a "pharmaceutical composition for intradermal administration", or a "pharmaceutical composition for administration by intradermal route", comprising at least one allergoid and at least one pharmaceutically acceptable excipient for intradermal administration of said allergoid. In the present description, said terms "pharmaceutical composition for intradermal administration", "pharmaceutical composition for administration by intradermal route" or "intradermal pharmaceutical composition" are used indistinctly with the same meaning.

An "allergoid" is a compound with very reduced allergen reactivity compared to the native allergen from which it is derived in terms of IgE allergen-specific binding, at the same time maintaining a high degree of other desirable properties, characteristic of the native allergen, including the ability to induce allergen-neutralizing IgG type antibody synthesis, protecting atopic individuals against post-exposure allergic symptoms and restoring the impaired immunity in allergic patients [Hans J Maasch & David G. Marsh, Standardized extract modified allergens allergoids. Clin Rev Allergy. (1987). 5: 89-106]. In other words, an allergoid maintains the immunogenicity of the native allergen but with an allergenicity that is less than that of the native allergen, so it can be used in immunotherapy (IT) because it can be administered at high doses with a low risk of systemic reactions.

Allergoids can be obtained from the corresponding allergens by methods known by persons skilled in the art. Although virtually any method can be used for obtaining an allergoid, in a preferred particular disclosure, said method is based on polymerization with glutaraldehyde [Patterson R. The Journal of Allergy and Clinical Immunology. (1981). Vol. 68(2):85-90; Maasch & Marsh, mentioned above]. Other methods use modification with formaldehyde [Bousquet J. et al. J Allergy Clin Immunol. (1989). 84:546-56], glutaraldehyde [Grammer LC et al. J Allergy Clin Immunol. (1985). 76:397-401] and alginate [Corrado OJ et al. Allergy. (1989). 44:108-15]. The European Medicines Agency (EMA) Guidelines, *"GUIDELINE ON ALLERGEN PRODUCTS: PRODUCTION AND QUALITY ISSUES"*, EMEA/CHMP/BWP/304831/2007 refer to allergoids in the "Definition" section (page 14) as allergens that are chemically modified to reduce IgE reactivity. The opinion paper issued by the World Health Organization (WHO) in 1998 entitled "Allergen Immunotherapy: Therapeutic Vaccines for Allergic Diseases", includes in section 2.3.3 glutaraldehyde-modified vaccines among the so-called allergoids. Therefore, glutaraldehyde polymerization is a chemical modification which is considered as such in section 4.2.4.3 of the aforementioned EMA guidelines. Therefore, an allergoid is a polymerized (i.e., chemically modified) allergen.

An allergen is any compound, substance or material capable of evoking an allergic reaction and inducing IgE synthesis. It is generally considered that allergens are a sub-category of antigens, which are compounds, substances or materials capable of evoking an immune response. Allergens can be natural or native, synthetic, recombinant, etc. In terms of their chemical or biochemical nature, allergens are usually native or recombinant proteins (or peptides), variants or fragments of said native or recombinant proteins (or peptides), fusion proteins, synthetic compounds (chemical allergens), synthetic compounds mimicking allergens, etc. Non-limiting illustrative examples of allergens include, among others, plant pollen allergens, animal epidermal derivative allergens, dust mite allergens, fungus allergens, food allergens, animal venom allergens and latex (*Hevea brasiliensis*) allergens.

In a particular disclosure, the allergoid present in the composition is an allergoid obtained from an individual plant pollen allergen or allergen extract, an allergoid obtained from an individual animal epidermal derivative allergen or allergen extract, an allergoid obtained from an individual dust mite allergen or allergen extract, an allergoid obtained from an individual fungus allergen or allergen extract, an allergoid obtained from an individual food allergen or allergen extract, an allergoid obtained from an individual animal venom allergen or allergen extract, or an allergoid obtained from an individual latex (*Hevea brasiliensis*) allergen or allergen extract.

In a particular disclosure, the allergoid present in the composition is an allergoid obtained from an individual plant pollen allergen or allergen extract, such as, for example, grasses, weeds, trees, undergrowth, etc.; non-limiting illustrative examples of such plants include Agropyron spp, *Acacia dealbata, Alnus glutinosa, Amaranthus spp, Ambrosia spp, Artemisia vulgaris, Avena sativa, Betula verrucosa, Chenopodium album, Chrysanthemum spp, Citrus sinensis, Corylus avellana, Cryptomeria* japonica, *Cupressus arizonica, Cupressus sempervirens, Cynodon dactylon, Dactylis glomerata, Eucalyptus spp, Fagus sylvatica, Festuca pratensis, Fraxinus excelsior, Helianthus spp, Hevea brasiliensis, Holcus lanatus, Hordeum vulgare, Jasminum spp, Juniperus oxycedrus, Ligustrum vulgare, Lolium perenne, Mercurialis annua, Morus alba, Olea europaea, Oryza sativa, Parietaria judaica, Phleum pratense, Phoenix canariensis, Phoenix dactylifera, Phragmites communis, Phytolacca dioica, Pinus sylvestris, Plantago lanceolata, Platanus acerifolia, Poa pratensis, Populus deltoides, Quercus ilex, Quercus robur, Quercus virginiana, Robinia pseudoacacia, Rumex acetosella, Salix nigra, Salsola kali, Sambucus nigra, Schinopsis sp., Secale cereale, Taraxacum officinale, Trisetum paniceum, Triticum aestivum, Ulmus campestris, Urtica dioica, Zea mays,* etc.

In a specific disclosure, said plant is *Phleum pratense.* In a more specific disclosure, the allergoid present in the composition is an allergoid obtained from an individual *Phleum pratense* allergen or allergen extract comprising one, two or more of the major and/or minor allergens described for *Phleum pratense,* for example, Phlp5, Phlp1, Phlp2, Phlp6, Phlp11, or Phlp12, etc.

In a particular disclosure, said plant is *Artemisia vulgaris* and the allergoid present in the composition is an allergoid obtained from an individual *Artemisia vulgaris* allergen or allergen extract. In another specific disclosure, said plant is *Parietaria judaica* and the allergoid present in the disclosed composition is an allergoid obtained from an individual *Parietaria judaica* allergen or allergen extract.

In a particular disclosure, the allergoid present in the composition is an allergoid obtained from an individual animal epidermal derivative allergen or allergen extract; non-limiting illustrative examples of such animals include *Canis familiaris, Equus caballus, Felis domesticus,* etc.

In a particular disclosure, the allergoid present in the composition is an allergoid obtained from an individual dust mite allergen or allergen extract; non-limiting illustrative examples of such mites include *Acarus siro, Blomia kulagini, Blomia tropicalis, Dermatophagoides farinae, Dermatophagoides pteronyssinus, Glycyphagus domesticus, Lepidoglyphus destructor, Tyrophagus putrescentiae,* etc. In a particular disclosure, said mite is *Dermatophagoides pteronyssinus* and the allergoid present in the disclosed composition is a *Dermatophagoides pteronyssinus* allergen extract. In a more specific disclosure, the allergoid present in the composition is an allergoid obtained from an individual *D. pteronyssinus* allergen or allergen extract comprising one, two or more of the major and/or minor allergens described for *D. pteronyssinus,* for example, Der p 1, Der p 2, Der p 3, Der p 6, Der p 10, or combinations thereof.

In a particular disclosure, the allergoid present in the composition is an allergoid obtained from an individual fungus allergen or allergen extract; non-limiting illustrative examples of such fungi include *Alternaria alternata, Aspergillus fumigatus, Aspergillus niger, Cladosporium herbarum, Penicillium notatum, Rhizopus nigricans,* etc.

In a particular disclosure, the allergoid present in the composition is an allergoid obtained from an individual food allergen or allergen extract; non-limiting illustrative examples of such foods include fish, milk and milk products (lactalbumin, lactoglobulin, casein, etc.), eggs and egg products (ovalbumin, ovomucoid, etc.), nuts and dried fruit (hazelnut, peanut, walnut, etc.), etc.

In a particular disclosure, the allergoid present in the composition is an allergoid obtained from an individual animal venom allergen or allergen extract; non-limiting illustrative examples of said animals include both insects, in any stage of development, for example, caterpillars, etc., and non-insect animals, for example, jellyfish, snakes, etc. In a specific disclosure, said insect is a hymenopter, for example, an insect belonging to the genus *Apis* (bees), an insect belonging to the family *Vespidae* (wasps), for example, of the genus *Polistes, Vespula,* etc., an insect belonging to the family *Formicidae* (ants), etc. In another specific disclosure, said insect is a diptera, for example, an insect belonging to the genus Aedes, *Culex, Culicoides,* etc. In another specific disclosure, said insect belongs to the order *Siphonaptera,* for example, an insect belonging to the genus *Ctenocephalides.* Non-limiting illustrative examples of said insects include *Aedes aegypti, Apis mellifera, Culex pipiens, Culicoides spp, Ctenocephalides sp., Formica fusca, Polistes sp., Vespula sp.,* etc. In a particular disclosure, said insect is *Apis mellifera* and the allergoid present in the disclosed composition is an allergoid of an *Apis mellifera* venom allergen extract. In a more specific disclosure, the allergoid present in the composition is an allergoid of an individual *Apis mellifera* venom allergen or allergen extract comprising one, two or more of the major and minor allergens described for *Apis mellifera* venom, for example, Api m 1, Api m 2, Api m 3, or their combinations.

In a specific disclosure, the allergoid present in the composition is an allergoid selected from the group consisting of allergoids obtained from an individual allergen or from an allergen extract of Agropyron spp, *Acacia dealbata, Alnus glutinosa, Amaranthus spp, Ambrosia spp, Artemisia vulgaris, Avena sativa, Betula verrucosa, Chenopodium album, Chrysanthemum spp, Citrus sinensis, Corylus avellana, Cryptomeria* japonica, *Cupressus arizonica, Cupressus sempervirens, Cynodon dactylon, Dactylis glomerata, Eucalyptus spp, Fagus sylvatica, Festuca pratensis, Fraxinus excelsior, Helianthus spp, Hevea brasiliensis, Holcus lanatus, Hordeum vulgare, Jasminum spp, Juniperus oxycedrus, Ligustrum vulgare, Lolium perenne, Mercurialis annua, Morus alba, Olea europaea, Oryza sativa, Parietaria judaica, Phleum pratense, Phoenix canariensis, Phoenix dactylifera, Phragmites communis, Phytolacca dioica, Pinus sylvestris, Plantago lanceolata, Platanus acerifolia, Poa pratensis, Populus deltoides, Quercus ilex, Quercus robur, Quercus virginiana, Robinia pseudoacacia, Rumex acetosella, Salix nigra, Salsola kali, Sambucus nigra, Schinopsis sp., Secale cereale, Taraxacum officinale, Trisetum paniceum, Triticum aestivum, Ulmus campestris, Urtica dioica, Zea mays, Canis familiaris, Equus caballus, Felis domesticus, Acarus siro, Blomia kulagini, Blomia tropicalis, Dermatophagoides pteronyssinus, Dermatophagoides farinae, Glycyphagus domesticus, Lepidoglyphus destructor, Tyrophagus putrescentiae, Alternaria alternata, Aspergillus fumigatus, Aspergillus niger, Cladosporium herbarum, Penicillium notatum, Rhizopus nigricans,* fish, milk and milk products (e.g., lactalbumin, lactoglobulin, casein, etc.), eggs and egg products (e.g., ovalbumin, ovomucoid, etc.), nuts and dried fruit (e.g., hazelnut, peanut, walnut, etc.), insects, for example, insects of the family *Formicidae,* etc., jellyfish venom, snake venom, caterpillar venom, etc., hymenoptera venoms, for example, insect venoms from insects of the genus *Apis* (e.g., *Apis mellifera*), insect venoms from insects of the family *Vespidae,* for example, insects of the genus *Polistes, Vespula,* etc., diptera venom, for example, insects belonging to the genera *Aedes, Culex, Culicoides,* etc.; insect venoms from insects belonging to the order *Siphonaptera,* for example, insects of the genus *Ctenocephalides,* etc., for example, venom of *Aedes aegypti, Apis mellifera, Culex pipiens, Culicoides spp, Ctenocephalides sp., Formica fusca, Polistes sp., Vespula sp.,* etc., latex (*Hevea brasiliensis*) allergens, or combinations thereof.

In a particular disclosure, the composition comprises a single allergoid. This particular disclosure is particularly useful for the treatment of an allergy to an allergen.

In another particular disclosure, the composition comprises two or more different allergoids. This particular disclosure is particularly useful for simultaneously treating two or more allergies to two or more different allergens.

The person skilled in the art will understand that allergies to various allergens can be simultaneously treated by means of the suitable formulation of the disclosed composition; to that end, the corresponding allergoids obtained by means of chemical modification, for example, by means of polymerizing the allergens in question with glutaraldehyde are simply included in the formulation of the disclosed composition. By way of non-limiting illustration, a composition including one or more allergoids obtained from individual grass pollen allergens or allergen extracts and/or one or more allergoids obtained from individual arizonica pollen allergens or allergen extracts could be formulated; said disclosed composition would be useful for the simultaneous treatment of an allergy to grass pollen and/or of an allergy to arizonica pollen. Any combination of allergoids such as those defined above can be used within the scope of the present invention.

In a particular disclosure, the composition is further characterized in that it is free of an immunological adjuvant. In another particular disclosure, the composition comprises one or more adjuvants.

As it is used herein, an immunological "adjuvant" is a substance (or combination of substances) used in combination with a specific allergen (particular case of an antigen) that produces a more robust immune response than the allergen alone. This definition includes a wide range of materials. Some immunological adjuvants that are used in many marketed vaccine products include mineral salts, particularly calcium phosphate, aluminum phosphate, etc., or bases, for example, aluminum hydroxide, etc. More effective immunostimulatory adjuvants include immunostimulatory oligodeoxynucleotides, immunostimulatory RNAs, proteins, including antibodies or small synthetic chemical entities that bind to immunostimulatory or co-stimulatory substance receptors, such as Toll-like receptors, for example. Non-limiting illustrative examples of adjuvants include saponins (such as QS21), cytokines (such as IL-2, IL-12), MDP derivatives, LPS, MLP and their derivatives, GM-CSF, lipopeptides and imiquimod.

The amount of allergoid in the disclosed composition will be selected based on the nature of the allergoid present therein; nevertheless in a particular disclosure, the allergoid content present in a unit dose of 0.1 mL is comprised between about 0.001 $\mu$g and about 1,000 $\mu$g of protein per dose. Preferably, said dose is the non-reactive maximum dose (end-point titer) of the allergoid administered by intradermal route in allergic subjects sensitized with the allergen. Said dose is a safe dose because it corresponds to the allergoid concentration that makes the skin test negative and can be determined by conventional methods known by persons skilled in the art (Example 6). In a particular disclosure, said non-reactive maximum dose of the allergoid of *Phleum pratense* [*Phleum pratense* allergen (protein) extract comprising the all or part of the described glutaraldehyde-polymerized *Phleum pratense* allergens] is 0.27 $\mu$g of protein extract/mL. Said value has been established in a single-center, open-label, native *Phleum pratense* extract-controlled clinical trial to obtain the non-reactive maximum dose (end-point titer) with said allergoid of *Phleum pratense* in patients with allergic rhinoconjunctivitis with or without intermittent mild or moderate asthma, sensitized to *Phleum pratense* pollen.

In a particular disclosure, the composition is formulated as a liquid formulation for intradermal administration. In another particular disclosure, the composition is formulated as a lyophilized powder formulation which, after reconstitution, leads to a liquid formulation for intradermal administration, for which purpose the excipients most suited to that end will be chosen. As it is used herein, the liquid formulation obtained after reconstituting the lyophilisate is characterized by the liquid state of at least the continuous phase of the composition. The liquid formulation can contain a single phase or, alternatively, it can incorporate one or more additional phases which are dispersed in the continuous phase and which may (or may not) be liquid. For example, a suspension is a liquid comprising a dispersed solid phase, and an emulsion is a liquid comprising a dispersed liquid phase. Excipients and techniques for formulating liquid compositions for intradermal administration are known to the person skilled in the art. Additional information about excipients suitable for formulating liquid formulations for intradermal administration can be found, for example, in "Tratado de Farmacia Galénica", C. Fauli i Trillo, 1993, Luzán 5, S.A. Ediciones, Madrid; and in Remington's Pharmaceutical Sciences (A.R. Gennaro, Ed.), 20th edition, Williams & Wilkins PA, USA (2000). Among the preferred excipients for the disclosed composition are physiological saline and mannitol. Optionally, one or more co-solvents can be incorporated. Depending on its chemical nature, the allergoid can be dissolved, colloidally dispersed or suspended (dispersed) in the liquid phase. In a particular disclosure, the allergoid is incorporated in a dissolved or colloidally dispersed state.

Optional additional excipients for the liquid formulation of the disclosed composition include pharmaceutically acceptable gelling agents, surfactants, co-surfactants, stabilizers, pH regulating agents such as acids, bases and buffer salts, preservatives, sugars, etc.

In a particular disclosure, the allergoid present in the composition is lyophilized.

In another particular disclosure, the allergoid present in the composition for use according of the invention is dissolved in an excipient suitable for administration by intradermal route. In a specific disclosure, said excipient is physiological saline and mannitol at 10 mg/mL.

In another particular disclosure, the composition is presented in single-dose form. In a specific embodiment, the disclosed composition is presented in single-dose form in which the allergoid content per unit dose of 0.1 mL is comprised between about 0.001 $\mu$g and about 1,000 $\mu$g of protein per dose. In a particular disclosure, said dose is the non-reactive maximum dose (end-point titer) of the allergoid administered by intradermal route in allergic subjects sensitized with the allergen. Said dose can be determined by conventional methods known by persons skilled in the art; by way of illustration, said dose can be established as a non-reactive dose in phase I diagnosis, advantageously as the non-reactive maximum dose in phase I diagnosis. In a particular disclosure, the composition comprises a dose of 0.27 $\mu$g of protein/mL of allergoid of *Phleum pratense,* wherein said allergoid was obtained from a glutaraldehyde-polymerized *Phleum pratense* allergen extract.

## Medical uses

In one aspect, the invention relates to a pharmaceutical composition for use in the treatment of a subject suffering from an allergy to an allergen, wherein the composition is to be administered intradermally, the composition comprising at least one allergoid and at least one excipient, wherein said allergoid is an allergoid obtained by means of chemically modifying said allergen, wherein said allergoid is present in the composition at the non-reactive maximum dose and wherein said non-reactive maximum dose corresponds to the allergoid concentration that, administered by the intradermal route, makes the skin test negative as tested on a population that is specifically sensitized to said allergen by means of an end-point titration study with the allergoid in a population of patients sensitized to the allergen.

In a particular embodiment, said allergoid of the composition for use according to the invention is an allergoid obtained by means of polymerizing said allergen with glutaraldehyde.

The features of the composition have already been discussed above and are incorporated herein by reference.

As it is used herein, the term "subject" includes any animal, preferably a mammal, including humans. Therefore, said method can be used for the treatment of allergies both in human beings and in animals, for example, dogs, cats, horses, etc.

The person skilled in the art will understand that in order for said immunotherapeutic treatment to be efficient, the allergoid present in the composition for use in the treatment of said allergy of the invention will be an allergoid obtained by means of chemically modifying the allergen to which the subject is sensitive. Therefore, once the allergen to which the subject is sensitive is known, an allergoid can be prepared according to the present invention by means of chemically modifying said allergen, preferably by means of polymerization with glutaraldehyde, and said allergoid can be formulated for administration by intradermal route to the subject by means of formulating it in a suitable pharmaceutical composition for intradermal administration.

In a particular embodiment, the composition to be used in the medical use indicated above comprises a single allergoid, so it is particularly useful for the treatment of the allergy to a single allergen, for example, allergy to *Phleum pratense* pollen, or allergy to *Artemisia vulgaris* pollen, or allergy to *Parietaria judaica* pollen, or allergy to *Dermatophagoides pteronyssinus,* or allergy to *Apis mellifera* venom, or allergy to dog or cat dander, etc.

In another particular embodiment, the composition o to be used in the medical use indicated above comprises two or more different allergoids; in this case, said composition is particularly useful for simultaneously treating two or more allergies to two or more different allergens. By way of non-limiting illustration, the invention contemplates the possibility of simultaneously treating the allergy to two or more different allergens, for example, allergies to two or more types of different pollens (e.g., *Phleum pratense* pollen, *Artemisia vulgaris* pollen, *Parietaria judaica* pollen, etc.); allergies to one or more plant pollens and to dust mites; allergies to one or more plant pollens and to insect venoms (e.g., *Apis mellifera*); allergies to dust mites and allergies to insect venoms; allergies to one or more plant pollens and to dog or cat dander; etc. The person skilled in the art will understand that by means of the suitable formulation of the composition for use according to the invention allergies to various allergens can be simultaneously treated. Therefore, once the allergens causing an allergy in a subject are identified, the person skilled in the art could formulate the suitable composition for use according to the invention, which will incorporate the allergoids corresponding to the allergens producing the allergic reaction in the subject to be treated. Any combination of allergoids such as those that have been defined above can be used within the scope of the present invention.

The dosage regimen of the composition for use according to the invention will be chosen depending on the severity of the disease or condition of the particular subject, the immunological sensitivity of the subject to the allergoid(s) comprised in the composition for use according to the invention, etc.

The administration of the composition for use according to the invention is typically performed with a frequency ranging from about once a day to once a year, preferably, once every two days, three times a week, two times a week, once a week, and once every two weeks.

The treatment will generally last for a time period comprised between about one week and about 6 months, or more preferably from about 2 weeks to about 5 months, for example about 1 month, about 6 weeks, about 2 months, or about 3 months, and in any case when the subject allergen tolerance is demonstrated.

For a treatment period, the administered dose can remain substantially constant or it can be adjusted.

To sustain the positive effects of a therapy that has been carried out for a period of several weeks or months, it may be useful to continue with a reduced administration frequency, such as about once every month, about once every 2 months, about once every 3 months, about once every 6 months or once a year.

Use

It is disclosed the use of a composition for preparing a medicinal product for the treatment of a subject's allergy to an allergen, wherein said composition is a pharmaceutical composition for intradermal administration comprising at least one allergoid and at least one pharmaceutically acceptable excipient, wherein said allergoid is an allergoid obtained by means of chemically modifying said allergen.

The features of the disclosed composition, the subject and the allergoid have already been discussed above. The person skilled in the art will understand that in order for the therapeutic use of the composition of the invention to be efficient, the allergoid present therein must be an allergoid of the allergen to which the subject is sensitive.

In a preferred particular embodiment, said allergoid is an allergoid obtained by means of polymerizing said allergen with glutaraldehyde.

In a particular embodiment, said composition for use according to the invention comprises a single allergoid. In another particular embodiment, the composition of the invention comprises two or more different allergoids.

It is also disclosed the use of an allergoid for preparing a pharmaceutical composition for intradermal administration to a subject for the treatment of said subject's allergy to an allergen, wherein said allergoid is an allergoid obtained by means of chemically modifying said allergen. It is also disclosed an allergoid for use in the treatment of a subject's allergy to an allergen by means of intradermal administration of said allergoid, wherein said allergoid is an allergoid obtained by means of chemically modifying said allergen.

The characteristics of the subject and the allergoid have already been discussed above. The person skilled in the art will understand that in order for the therapeutic use of the allergoid to be efficient in the treatment of an allergy to an allergen, said allergoid must be an allergoid of the allergen to which the subject is sensitive.

In a preferred disclosure, said allergoid is an allergoid obtained by means of polymerizing said allergen with glutaraldehyde.

In a particular disclosure, said pharmaceutical composition comprises a single allergoid. In another particular disclosure, said pharmaceutical composition comprises two or more different allergoids.

Kit

It is disclosed a pharmaceutical kit, comprising the disclosed composition and the means and instructions for administering said composition, wherein said composition is a pharmaceutical composition for intradermal administration comprising at least one allergoid and at least one pharmaceutically acceptable excipient.

The features of the composition and of the allergoid have already been discussed above and are incorporated herein by reference. In a particular disclosure, said pharmaceutical composition comprises a single allergoid. In another particular disclosure, said pharmaceutical composition comprises two or more different allergoids.

In a particular disclosure, said allergoid is lyophilized and the kit comprises a variable number of vials, typically from 1 to 4 vials, with an excipient suitable for administration of the allergoid by intradermal route after reconstitution.

The instructions can also comprise any of the optional or preferred features of the medical use described previously.

Advantageously, the kit of the disclosure can comprise not only the composition and the instructions, but also the means for administration, particularly the needles suitable for intradermal administration of the disclosed composition, the necessary vials and other devices aiding with the intradermal administration of the allergoid.

According to the present invention, the pharmaceutical composition of the invention and the kit of the invention are for use in the treatment of subjects suffering from an allergy to an allergen.

Thus, the invention relates to a pharmaceutical kit comprising a pharmaceutical composition wherein the composition is to be administered intradermally, the composition comprising at least one allergoid and at least one excipient, wherein said allergoid is an allergoid obtained by means of chemically modifying said allergen, wherein said allergoid is present in the composition at the non-reactive maximum dose and wherein said non-reactive maximum dose corresponds to the allergoid concentration that, administered by the intradermal route, makes the skin test negative as tested on a population that is specifically sensitized to said allergen by means of an end-point titration study with the allergoid in a population of patients sensitized to the allergen and the means and instructions for administering said composition for use in the treatment of a subject suffering from an allergy to an allergen.

As it is used herein, the term "allergy" includes any form of latent or manifest type-I hypersensitivity, also known as immediate or IgE-mediated hypersensitivity. Type-I hypersensitivity is characterized by the activation of mast cells and basophils through immunoglobulin E (IgE) which leads to the release of the mediators contained in their granules, causing a systemic inflammatory response that can cause a wide range of mild, moderate and severe symptoms ranging from a runny nose (rhinorrhea) to a life-threatening anaphylactic shock. The fundamental difference between a type-I hypersensitivity reaction to an allergen and a normalized humoral response to said allergen is that in hypersensitivity, the plasma cells predominantly secrete type E instead of type G immunoglobulins, which are secreted against the recognized antigens.

Allergic diseases also can be classified according to the predominant symptoms with which they are associated. For example, the invention comprises the treatment of subjects suffering from seasonal allergic rhinitis (hay fever), perennial allergic rhinitis, allergic conjunctivitis, and combinations thereof, such as, for example, rhinosinusitis, asthma, atopic dermatitis, urticaria, as well as the treatment of any food allergy, house dust allergy, or an allergy to any of the allergens herein mentioned.

The scope of the invention extends to the treatment of any allergic subject, without restrictions.

In an additional preferred embodiment, the medical use of the invention is used for the treatment that establishes the impaired normal immune response of allergic patients, as defined by the WHO, of those subjects having a manifest allergic disease or who are at a higher risk of developing an allergy or a more severe allergic pathology, as demonstrated in the transition from rhinitis to asthma.

The intradermal administration of allergoids according to the present invention has a number of advantages because:

1. The intradermal application of allergoids allows obtaining an immunogenic effect, as shown by means of the immunization studies performed in rabbits, but with a reduced allergenicity of the allergen extract, as shown by means of the histamine release and $Ag_{50}$ value determination.

2. The reduction of allergenicity and of the volume to be administered, maximum 0.1 mL of the polymerized allergen extract, together with the intradermal administration route, associated with the use of the allergoids, stresses the increase in the safety of the vaccine preparations.

3. It allows eliminating the escalation dose to reach the maximum dose tolerated by the patient, given that in the design of the allergoid a single administration dose, established in terms of safety is considered as the allergoid dilution that makes the intradermal skin test performed with it negative. The invention shows that this dose is safe given that diagnosis and immunotherapy are performed through the same intradermal route, and it is therefore possible to establish for each allergoid the maximum tolerated dose depending on patient sensitivity since it is tested on a population that is specifically sensitized to the allergen in question. Therefore, the intradermal application allows establishing the maximum safe and effective dose of an allergoid, considering the difference in sensitivity between subjects (patients) and allowing the adjustment thereof based on the dose escalation established in the diagnosis until being able to fix said dose.

4. The optimal dose - tolerated dose of allergoid concept can be tested by means of intradermal (ID) skin tests in a population sensitized to an allergen by means of end-point titration and suitably established because administration of the immunotherapy (IT) uses the same diagnostic route for each allergen.

5. It allows eliminating the diversity of the volumes to be administered in allergen extract immunotherapy, which is a factor that gives rise to the possibility of inducing overdose reactions in clinical practice (the maximum volume to be administered will always be 0.1 mL, given that this limit is inherent to the intradermal administration technique). 6. It allows maintaining allergoid stability and activity since it contemplates the possibility of being designed in the form of a lyophilized powder with a cryoprotectant (e.g., mannitol) and minimizing to the greatest extent possible the possible variations and losses of biological activity.

The present invention for the first time demonstrates the possibility of using the intradermal route with allergoids for therapeutic purposes. The use of intradermal tests with allergens for diagnostic purposes (common practice in the United States for establishing the etiological diagnosis) has historically been common practice until now.

Since the invention considers the intradermal administration route of allergoids, it represents an innovation that has not been tested before. The safety and efficacy of the intradermal route has been tested and widely demonstrated with other immunizing agents (e.g., BCG vaccine, rabies vaccines, and recently the flu vaccine). This administration route allows improving antigen processing and recognition given the wide distribution of antigen presenting cells, and therefore the role as an immune response modulator.

The invention is additionally illustrated by means of the following examples.

EXAMPLE 1

General protocol for obtaining pollen allergen extracts

1.1 Obtaining pollen allergen extracts

The pollen raw material is acquired from a producer, such as, for example, Allergon, Pharmalerga, etc., and, once received it is stored in the cold chamber until it is processed. Alternatively, the pollen raw material is supplied from the pollen collection that the applicant has.

The pollen is weighed in a sterile container, the amount of raw material to be extracted is recorded and is poured into a beaker under a laminar flow hood. Water-soluble extraction is performed by maceration with phosphate buffer saline (PBS), by means of gentle magnetic stirring in an agitator (at about 1000 rpm) and at a temperature comprised between 2°C and 8°C (cold chamber) for 4 hours. The pH of the extract is maintained at a value of 7.5-8.5 with 1N NaOH or HCl by means of measuring in a pH meter.

After this step, the extract is subjected to centrifugation at 5,000 rpm for 30 minutes in a centrifuge and the supernatant is passed through clarifying filters, conductivity being measured with a conductivity meter and storing the resulting supernatant at a temperature comprised between 2°C and 8°C.

The centrifugation sediment is resuspended in PBS. This second extract is subjected to centrifugation at 5,000 rpm for 30 minutes, the sediment being discarded and pooling the supernatant with the supernatant obtained in the previous centrifugation to continue processing it. The obtained supernatant is passed through clarifying filters and its conductivity is measured.

Both filtered supernatants are subsequently mixed together, and the crude extract is subjected to concentration by tangential flow ultrafiltration in suitable equipment, for example a Cogent M1 system (Millipore) equipped with a system of packed membranes (Cassette) with a size exclusion limit of 5,000 Daltons (Da) [3,000 Da if processing *Parietaria judaica, Artemisia vulgaris* and *Ambrosia trifida,* for example]. Dialysis is performed by adding 5 volumes of injection-grade water with respect to the concentrated volume. When the final volume of the obtained crude pollen extract is less than 600 mL, dialysis is performed in Labscale by adding 5 volumes of WFI water with respect to the obtained volume using 5 kDa cartridges. Conductivity is measured with a conductivity meter at the end of the process, and said conductivity must be less than 1.5 mS.

The extract is then aliquoted into vials at a proportion of 30 mL/vial which are stoppered and subsequently frozen at -20°C (if they are not immediately lyophilized).

For lyophilization, the vials are introduced in a sterile chamber in a lyophilizer, for example, a Telstar lyophilizer. At the end of the lyophilization cycle, the content of 1 vial is weighed to calculate the yield, expressed in grams of lyophilized intermediate product per 100 grams of raw material. Finally, the obtained product and the documentation are delivered to Quality Control for characterization.

A native *Phleum pratense* allergen extract that was used in Examples 4 and 5 was obtained following the protocol indicated above.

1.2 Characterization of the native allergen extract

1.2.1 Polyacrylamide gel electrophoresis

Buffers and solutions

Acrylamide/bisacrylamide, 1.5 M Tris-HCl, pH 8.8; 0.5 M Tris-HCl, pH 6.8 (Stacking); 10% sodium dodecyl sulfate (SDS) solution; 30 mg/mL ammonium persulfate; Temed: (N,N,N',N'-tetramethylethylenediamine); 5X sample buffer; electrode buffer, pH 8.3; molecular weight standard; silver fixer; silver accelerator; silver stain (pure water, silver complex solution, reduction moderator solution and image development reagent). Coomassie (staining) solution; Coomassie destaining solution.

Method

The gels used are prepared with the following proportions of acrylamide:separating gel 12.5% and stacking gel 4%.
15 $\mu$g of protein (allergen extracts)/lane are loaded for Coomassie staining (10 $\mu$L of a sample diluted to 1.5 mg protein/mL are loaded) and 0.5 $\mu$g of protein/lane are loaded for silver staining (10 $\mu$L of a sample diluted to 0.05 mg protein/mL are loaded).
Once the samples are diluted to the protein concentration necessary for the type of staining that is going to be used, the samples are then prepared. To that end, 80 $\mu$L of the previously obtained dilution and 20 $\mu$L of 5X sample buffer (4:1 ratio) are taken in an Eppendorf tube and the mixture is heated for 8 minutes at about 100°C. After cooling (room temperature), the insoluble residues are removed by centrifuging for 5 minutes at 14,000 rpm.

Electrophoretic conditions

The following are dispensed:

- in one lane, 7 $\mu$L of molecular weight standard for Coomassie staining, or 4 $\mu$L of molecular weight standard for silver staining;
- in one lane, 10 $\mu$L of IHR (in-house reference) for the case of standardized allergen extracts (for the case of non-standardized allergen extracts this lane will be omitted); and
- in three lanes, 10 $\mu$L of allergen extract to be analyzed.

When the migration front is close to the end of the gel, at about 45 minutes after having started electrophoresis, the source is disconnected and the gel is removed. The gel is subsequently stained and destained depending on the protein concentration each sample has:

- Coomassie staining: the gel is placed on a 10 cm x 10 cm plate and the Coomassie solution is added. Staining: 1 hour at 60°C, 2 hours at 37°C, or 12 hours at room temperature.
- Coomassie destaining solution: the Coomassie destaining solution is added until the bottom of the gel is almost transparent and the bands of the standard and the samples can be seen well. Once it is completely decolorized, the solution is disposed of and water is added so that it is in optimal hydration conditions.

The molecular weights are determined by the Weber and Osborn method [Weber, K. & Osborn, M. J. Biol. Chem. 244:4406-4412 (1969)], by constructing a calibration line, representing the mobility (in mm) manifested by a series of proteins with respect to the logarithm of their molecular weight (known). Mobility is measured from the start of the separating gel to the midpoint of the band. Once the correlation between both variables (r> 0.90) is demonstrated, the molecular weights are determined with a computer program (Diversity database) based on the Weber and Osborn method. The molecular weights of the proteins used as standards are: 250, 150, 100 and 75 Daltons.

1.2.2 Determining the protein

Reagents and preparation

The reading is performed at 595 nm in a spectrophotometer (for example, a BECKMAN COULTER DU 640 spectrophotometer), with a quartz cuvette for use in the visible spectrum. Concentrated BIO-RAD reagent (Protein assay. Ref: 500-0006) is used to perform the assay in order to determine the protein content, said reagent being prepared at a proportion of 0.2 mL of BIO-RAD reagent per mL of reagent (e.g., 20 mL BIO-RAD reagent + 80 mL pure water). A standard curve is prepared starting from a bovine serum albumin (BSA) stock solution at 1 mg/mL. To that end, various concentrations are prepared: 2; 4; 6; 8 and 10 μg/mL. This curve is prepared in triplicate in a 1.5 mL Eppendorf tube, with a final volume of 1 mL to be completed with the reagent. 1 mL of the already diluted reagent, which will previously be read in the spectrophotometer, is used as a blank.

### Preparation of the samples

This preparation begins by preparing 900 μL aliquots of reagent prepared in a 1.5 mL Eppendorf tube up to a total of 6 per sample.

### Acceptance limits

The obtained absolute value relating to the amount of protein contained in the sample must be at ± 50-150% with respect to the IHR batch (according to Eur. Ph. monograph EMEA/BWP/304831/2007).

### 1.2.3 ELISA inhibition with human serum

The products to be analyzed (about 5-6 mg) are weighed on a scale. The weighed samples are reconstituted at 10 mg/mL with pure water, shaken and centrifuged for 5 minutes at 14,000 rpm.

A solution of the reference extract (IHR) to be analyzed is prepared in 50 mM carbonate/bicarbonate buffer, pH 9.6 at a concentration of 250 μg/mL (6 mL of carbonate/bicarbonate buffer and 150 μL of 10 mg/mL IHR to be analyzed).

50 μL of IHR solution (250 μg/mL) are pipetted into the 96 wells of the ELISA plate and the plate is covered to prevent evaporation. It is incubated overnight at a temperature comprised between 2°C and 8°C in a refrigerator.

The ELISA plate is washed with 0.05% PBS-Tween® 20, pH 7.4, in a Biotek Elx50 washer. It is then blocked by adding 200 μL/well of 1% BSA in 0.05% PBS-Tween® 20 and incubated at room temperature for 1 hour.

Preparation of the samples to be analyzed: The IHR and active substances to be analyzed are prepared in an Eppendorf tube at 1 mg/mL with 1% BSA in 0.05% PBS-Tween® 20. 90 μL of the previously prepared dilutions of the products to be analyzed plus 90 μL of the corresponding human serum are added to another Eppendorf tube.

To prepare the positive control (BSA + human serum), 90 μL of 1% BSA in 0.05% PBS-Tween® 20 (instead of the sample) plus 90 μL of the corresponding human serum are added to an Eppendorf tube.

To prepare the negative control (BSA), 180 μL of 1% BSA in 0.05% PBS-Tween® 20 are added to an Eppendorf tube.

The samples are incubated together with the positive control and the negative control for 1 hour at room temperature and while being rotated. After the incubation time of the samples has elapsed, they are centrifuged for 10 seconds at 14,000 rpm. 50 μL of the samples are pipetted into an ELISA plate and the plate is incubated for 2 hours at room temperature. After the blocking time (2 hours) has elapsed, the ELISA plate is washed with 0.05% PBS-Tween® 20, pH 7.4. 50 μL/well of E-27-IgG Human IgE monoclonal antibody (operon) at a dilution of 1/1000 are added and it is incubated for 30 minutes at room temperature. The plate is washed with 0.05% PBS-Tween® 20, pH 7.4. Then 50 μL/well of murine anti-IgG (γ chain-specific) at a dilution of 1/500 are added and it is incubated for 30 minutes at room temperature. The wells are washed with 0.05% PBS-Tween® 20, pH 7.4. Subsequently, 50 μL/well of the streptavidin-peroxidase conjugate of *Streptomyces avidinii* at a dilution of 1:250 (24 μL + 6 mL of 1% BSA in 0.05% PBS-Tween® 20) are added and it is incubated for 30 minutes at room temperature. The wells are washed with 0.05% PBS-Tween® 20, pH 7.4. Next, 50 μL/well of $H_2O_2$ + ABTS (2,2'-azino-bis(3-ethylbenzothiazoline-6-sulfonic acid) at a dilution of 1:1000 (6 μL of $H_2O_2$ + 6 mL of ABTS) are added. The plate is read using a Multiskan Ascent V1.23 plate reader.

### Calculating and presenting the results

Percentage of binding: A certain percentage of IgE bound to the bottom of the ELISA plate, with respect to 100% binding, corresponding to the positive control wells, without allergen (BSA + antibody). It is obtained by multiplying the average value of absorbance (Abs) for each allergen concentration by 100, and by dividing said product by the average absorbances of the positive control, according to the equation:

```
% binding  = mean Abs at each conc x 100/mean Abs +Control
```

Percentage of inhibition: Percentage of antibody not bound to the bottom of the plate corresponding to total antibody minus bound antibody. This value is calculated as:

$$\% \ inhibition = 100 - \% \ binding$$

Coefficient of correlation of the curve, which must be greater than or equal to 0.980.

Log Ag50 and Ag50 value: these correspond with the mean allergen concentration that produces 50% inhibition of IgE binding to the plate.

The LogAg50 value is obtained by extrapolating the different percentages of inhibition with respect to the different allergen concentration logarithms to calculate the allergen concentration capable of causing a 50% inhibition.

The Ag50 value is obtained by calculating the antilogarithm of the LogAg50 value. This value serves to compare allergen extracts to one another, as well as a preparation, treatment or diagnosis with respect to their IHR.

Acceptance limits: The acceptance limits of Ag50 of a specific assay are comprised between 50%-150% of the Ag50 value of the reference extract (IHR). These limits are established by the current ICH.

1.2.4 *Phleum pratense* Phlp5 ELISA

This assay starts with a 2 mg/mL stock solution of anti-Phlp5 monoclonal antibody (1D11) in PBS. 2,000 ng/well of 1D11 monoclonal antibody, diluted in 50 mM carbonate-bicarbonate buffer, pH 9.6 (10 $\mu$L of anti-Phlp5 monoclonal antibody in 10 mL of 50 mM carbonate-bicarbonate buffer, pH 9.6) are pipetted into a 96-well polystyrene microtiter plate, and are left to incubate overnight at 4°C. After that time has elapsed, the wells are washed with 0.05% PBS-Tween® 20, pH 7.4.

The plate is incubated for 30 minutes with 100 $\mu$l of 1% BSA in 0.05% PBS-T (PBS with Tween® 20).

To prepare the samples in which the major allergen Phlp5 is going to be quantified, preparation starts with a 10 mg/mL stock solution, both in the IHR and in the test samples, taken to a concentration of 1 mg/mL, using 0.05% PBS-T-1% BSA as a diluent. 1/200, 1/400 and 1/800 dilutions in 0.05% PBS-T-1% BSA are prepared from each dilution of product to be analyzed at 1 mg/mL.

At the same time a standard curve is prepared. The Universal allergen Standard rPhlp5a is used for that purpose, this standard being at a concentration of 5,000 ng/mL (Indoor Biotechnologies). In the case of the curve for rPhlp5, it has to cover a range from 250 to 0.98 ng/mL of rPhlp5a, at dilutions of 1:2 from one concentration to another. 10 $\mu$L of rPhlp5a (5,000 ng/mL) are pipetted directly into the wells on 190 $\mu$L of 1% BSA in 0.05% PBS-T previously added to the well. After 30 minutes of incubation in 1% BSA, the wells are washed with 0.05% PBS-Tween® 20, pH 7.4. The treatment of the plate when adding the Universal allergen Standard rPhlp5a comprises adding to each well, following the scheme established by the operator, 100 $\mu$L of allergen sample diluted to different dilutions of both the IHR and the test sample, and the plate is incubated at room temperature for 1 hour. After that incubation time in 0.05% PBS-T-1% BSA has elapsed, the wells are washed with 0.05% PBS-Tween® 20, pH 7.4. The plate is incubated for 1 hour with 100 $\mu$L of biotinylated antibody Bo1 (Indoor Biotechnologies) previously diluted to a dilution of 1/1000 in 0.05% PBS-T-1% BSA, i.e., 10 $\mu$L of biotinylated antibody Bo1 in 10 mL of 0.05% PBS-T-1% BSA are used. This monoclonal antibody is used as secondary antibody. After that incubation time in 0.05% PBS-T-1% BSA has elapsed, the wells are washed with 0.05% PBS-T, pH 7.4. The streptavidin-peroxidase conjugate of *Streptomyces avidinii,* at a dilution of 1:1000 in blocking solution (10 $\mu$L + 10 mL of 0.05% PBS-T-1% BSA), is subsequently added at a proportion of 100 $\mu$L per well and is incubated for 30 minutes at room temperature. After that incubation time in 1% BSA has elapsed, the wells are washed with 0.05% PBS-Tween® 20, pH 7.4.

100 $\mu$L of $H_2O_2$ + ABTS at a dilution of 1:1000 (10 $\mu$L of $H_2O_2$ + 10 mL ABTS) per well are added to develop the plate. The plate is read in a Multiskan Ascent V1.23 plate reader at 405 nm.

The acceptance limits of the major allergen concentrations of a specific assay are 50%-200% of the value of major allergens of the reference extract (IHR). These limits are established by the European Pharmacopoeia and US Pharmacopoeia.

EXAMPLE 2

Obtaining a glutaraldehyde-polymerized *Phleum pratense* pollen allergen extract

In a Eurotubo® sterile IVD 0.4 g of a lyophilized *Phleum pratense* allergen extract (350 mg protein/g of extract) comprising at least Phlp1, Phlp2, Phlp5, Phlp6, Phlp11 and/or Phlp12 are weighed and diluted in phosphate buffer saline (PBS), pH 7.4, at a concentration between 0.1 and 3 mg/mL, typically 1.4 mg/mL of protein of the *Phleum pratense* allergen extract (100 mL). Then, between 0.1 and 2 mL, typically 0.95 mL, of glutaraldehyde are added drop-wise to a concentration between 0.01 M and 0.3 M, typically 0.025 M, with gentle stirring (80 rpm) in a magnetic stirrer in a cold chamber at a temperature comprised between 2°C and 8°C for 24 hours, whereby obtaining a crude polymerized allergen extract.

The crude polymerized *Phleum pratense* allergen extract is subjected to tangential flow ultrafiltration (UF) (Labscale® Millipore) through a 300 KDa size exclusion cartridge (Pellicon® XL Millipore Filter), 10 volumes of injection-grade water with respect to the volume of crude polymerized *Phleum pratense* allergen extract, specifically 1,000 mL, being passed through. The resulting polymerized *Phleum pratense* allergen extract contains glutaraldehyde-polymerized proteins, with a molecular weight greater than 100,000 Da. The protein concentration present in the native allergen starting extract can be determined as indicated in Example 1.

EXAMPLE 3

Obtaining vials for treatment with glutaraldehyde-polymerized *Phleum pratense* allergen extract

Once the glutaraldehyde-polymerized *Phleum pratense* allergen extract subjected to tangential flow UF obtained in Example 2 is dialyzed in a Labscale® tangential flow UF system, it is subjected to filtration and the concentration of said polymerized allergen extract is adjusted as described below.

To carry out the sterilizing filtration of said glutaraldehyde-polymerized *Phleum pratense* allergen extract subjected to tangential flow UF, the 0.22 micrometers (μm) polyethersulfone sterilizing filter is previously activated. 2 mL of mannitol (5 mg/mL) are added to the filter and it is left to incubate for 5 minutes; then the vacuum system valve is opened until all the buffer (mannitol in water for injection at 5 mg/mL) is filtered, and the vacuum system valve is subsequently closed. This process is repeated once more. Then the filter is left to dry for 30 minutes (15 minutes with an open vacuum and another 15 minutes with a closed vacuum), 2.5 mL of the sample to be filtered (glutaraldehyde-polymerized *Phleum pratense* allergen extract subjected to tangential flow UF) are added and the vacuum valve is opened for 10 minutes until the filter dries, this step being repeated 2 more times. The filter is finally left to dry for 10 minutes.

Once the 0.22 μm polyethersulfone sterilizing filter is activated, the glutaraldehyde-polymerized *Phleum pratense* allergen extract subjected to tangential flow UF is subjected to sterilizing filtration. To that end, before filtering said glutaraldehyde-polymerized *Phleum pratense* allergen extract subjected to tangential flow UF, a 2 mL sample is taken to analyze the biological load of the product. Then the vacuum is opened and the glutaraldehyde-polymerized *Phleum pratense* allergen extract subjected to tangential flow UF is added to be filtered and once filtered, it is passed to a sterile chamber through the SAS system [Security Air System]. A 10 mL aliquot of said glutaraldehyde-polymerized *Phleum pratense* allergen extract subjected to tangential flow UF and sterilizing filtration is analyzed by means of the Bradford assay [Bradford, M.M. (1976), Anal. Biochem. 72: 248-254] to determine the amount of protein present in said extract and to subsequently perform the bulk packaging. The glutaraldehyde-polymerized *Phleum pratense* allergen extract subjected to tangential flow UF and sterilizing filtration is diluted in cryoprotective diluent (mannitol at 10 mg/mL) to obtain a final concentration comprised between 0.001 and 1.000 μg of protein per milliliter (mL) in the glutaraldehyde-polymerized *Phleum pratense* allergen extract subjected to tangential flow UF and sterilizing filtration. After adjusting to protein treatment concentration the obtained bulk product is metered into 2 mL capacity vials at a proportion of 1 mL/vial. Once the vials are metered, they are lyophilized by introducing them in a Telstar lyophilizer and performing a complete lyophilization cycle, which ends by vacuum sealing of the vials.

EXAMPLE 4

Study of the immunogenicity to native and glutaraldehyde-polymerized *Phleum pratense* pollen allergen extract

This assay was performed in collaboration with the Department of Immunology, Microbiology and Parasitology, of the School of Pharmacy (Basque Country University) to study the immunogenicity to native and glutaraldehyde-polymerized *Phleum pratense* allergen extracts.

To that end, native *Phleum pratense* allergen extract (*nPh.p*) or glutaraldehyde-polymerized *Phleum pratense* allergen extract (*pPh.p*) antisera were obtained by means of subcutaneous (SC) or intradermal (ID) inoculation of said extracts in 10 New Zealand albino rabbits with an initial weight of about 2.5 kg according to Table 1.

Table 1

| *Phleum pratense* allergen extract | | | |
|---|---|---|---|
| Subcutaneous route | | Intradermal route | |
| Native | Polymerized | Native | Polymerized |
| 2 rabbits | 2 rabbits | 2 rabbits | 2 rabbits |

(continued)

| Phleum pratense allergen extract | |
|---|---|
| Subcutaneous route | Intradermal route |
| | |
| Negative controls (mannitol) | |
| Subcutaneous route | Intradermal route |
| 1 rabbit | 1 rabbit |

Inoculation

- Subcutaneous inoculation (SC): the inocula for SC administration were prepared by diluting the vials containing (i) native *Phleum pratense* allergen extract (10 µg in 1 mL of 0.2% phenolated physiological saline solution with aluminum hydroxide as an adjuvant), obtained according to the protocol described in Example 1, applied to obtaining a native *Phleum pratense* allergen extract, or (ii) a glutaraldehyde-polymerized *Phleum pratense* allergen extract subjected to tangential flow UF and sterilizing filtration ("allergoid") -according to Examples 2 and 3-(10 µg of allergoid and 10 mg of mannitol in 1 mL of 0.2% phenolated physiological saline solution and with an adjuvant), 0.5 mL per dose being injected.
- Intradermal inoculation (ID) : the inocula for the ID administration of (i) native *Phleum pratense* allergen extract (10 µg) [obtained according to the protocol described in Example 1, applied to obtaining native *Phleum pratense* allergen extract] or (ii) glutaraldehyde-polymerized *Phleum pratense* allergen extract subjected to tangential flow UF and sterilizing filtration -according to Examples 2 and 3- (10 µg of allergoid and 10 mg of mannitol were reconstituted in 0.2 mL of 0.2% phenolated physiological saline solution), 0.1 mL per dose being injected.

Immunization regimen

Immunizations were carried out for 7 weeks after which final bleeding of the animal was performed, after titrating the serum, the results of which are expressed below in tables and figures.

Results

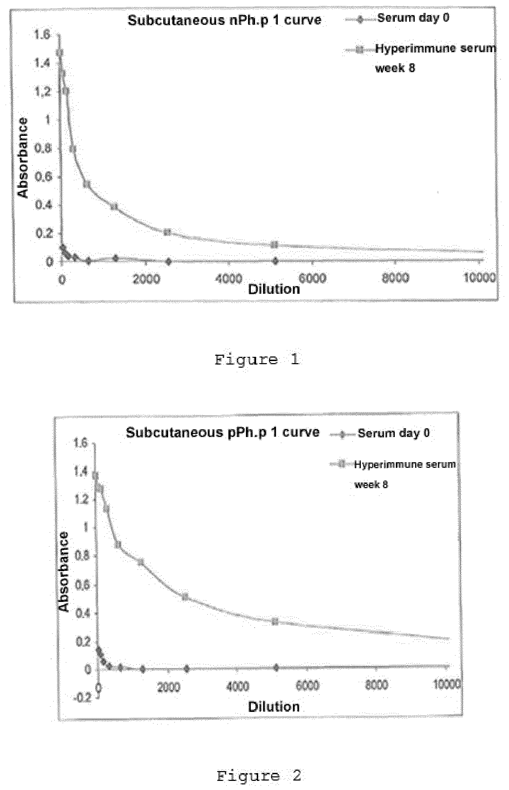

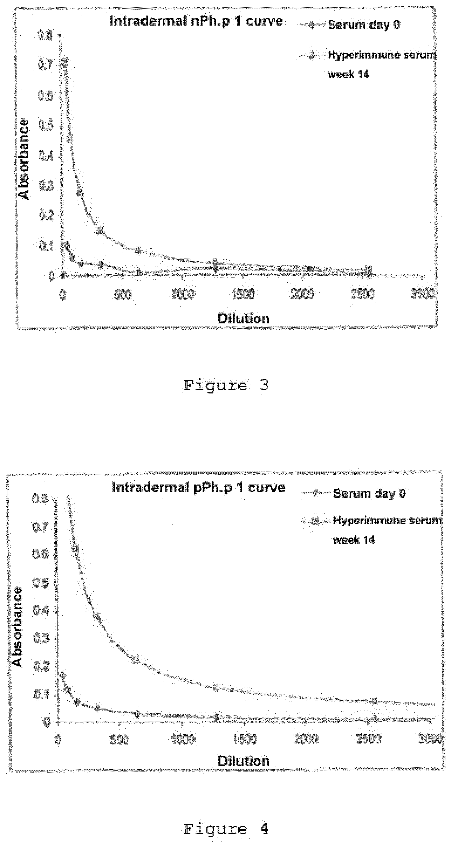

The obtained results are specified in figures shown below (Figures 1-4). Generally, the titration of the antisera (hyperimmune sera) obtained from the rabbits showed that the titers obtained by both administration routes (SC and ID) for the native and glutaraldehyde-polymerized *Phleum pratense* pollen allergen extracts showed similar titration curves and that the SC route allowed obtaining larger titers (maximum 1:1 - 1:2000) in less time (7 weeks) than the ID route (maximum 1:1 - 1:1000 in 14 weeks), although the volumes of *Phleum pratense* allergen extract administered per dose were 0.5 mL for the SC route and 0.1 mL for the ID route, and that aluminum hydroxide was used in the SC route as an adjuvant.

EXAMPLE 5

Reduction of allergenicity (Ag50 values and histamine release)

5.1 Ag50 values

Example 1 describes a protocol for obtaining and characterizing a native *Phleum pratense* pollen allergen extract from which a glutaraldehyde-polymerized *Phleum pratense* pollen allergen extract such as the one described in Examples 2 and 3 could be obtained.

Assays performed with said glutaraldehyde-polymerized *Phleum pratense* allergen extract according to Examples 2 and 3 have clearly shown the presence of all the allergens and their isoforms described by means of HPSEC (High-Performance Size Exclusion Chromatography); the polymerization process also shows the absence of IgE fixing bands in the molecular weight (MW) ranges of the allergens described by means of immunofixation and ELISA inhibition techniques showing the reduction of the IgE fixing capacity of the polymerized extract vs. the native extract in terms of

16

relative potency establishing a loss of said potency for the polymerized extract greater than 97%.

Table 2 shows the losses of potency (Ag50 values) of the three validated batches of glutaraldehyde-polymerized *Phleum pratense* allergen extract subjected to tangential flow UF and sterilizing filtration according to Examples 2 and 3.

Definition of Ag50: Ag50 is defined as the allergen extract concentration inhibiting 50% of the specific IgE present in the serum by means of enzyme-linked immunosorbent assay or radioimmunoassay techniques. The technique consists of assessing the potency of an allergen extract with respect to a serum rich in IgE specific to a certain allergen. The competition of IgE for the fixed allergen or allergen in solution is determined as a function of the so-called Ag50 or concentration of the allergen inhibiting 50% of RAST or ELISA. In general terms, it can be defined that the more an allergen extract inhibits RAST or ELISA the more potent it is, i.e., the lower the concentration of the allergen extract inducing 50% inhibition (Ag50 value) the more potent it is.

Table 2

| Polymerized *Phleum*: Ag50 | Native *Phleum*: Ag50 | Loss of potency |
|---|---|---|
| Phl p 003p-11: 16.90 $\mu$g/mL | Phl p 002-10: 0.18 $\mu$g/mL | 98.9% |
| Phl p 005p-11: 22.87 $\mu$g/mL | Phl p 002-10: 0.48 $\mu$g/mL | 97.8% |
| Phl p 006p-11: 16.04 $\mu$g/mL | Phl p 002-10: 0.37 $\mu$g/mL | 97.7% |

Polymerized *Phleum:* Glutaraldehyde-polymerized *Phleum pratense* allergen extract subjected to tangential flow UF and sterilizing filtration (Examples 2 and 3).

Native *Phleum:* Native *Phleum pratense* allergen extract [this extract can be obtained following the protocol described in Example 1].

The relative potency (RP), defined based on the determination of the Ag50 established for native *Phleum pratense* allergen extract vs. glutaraldehyde-polymerized *Phleum pratense* allergen extract subjected to tangential flow UF and sterilizing filtration, is calculated according to the following formula:

```
RP = Polymerized Phleum Ag50 value/Native Phleum Ag50 value
```

where

- "Polymerized *Phleum* Ag50 Value" refers to the Ag50 established for glutaraldehyde-polymerized *Phleum pratense* allergen extract subjected to tangential flow UF and sterilizing filtration; and
- "Native *Phleum* Ag50 value" refers to the Ag50 established for native *Phleum pratense* allergen extract.

The obtained RP value is thus 18.0603/0.343 = 52.654, which results from dividing the polymerized *Phleum* Ag50 value (mean of 3 batches) by the native *Phleum* Ag50 value (mean of 3 batches), according to the values indicated in Table 2.

5.2 Histamine release

The allergic patient basophil histamine release assay used is an approved method for evaluating the allergenicity of allergenic preparations [Siraganian RP: An automated continuous flow system for the extraction and fluorometric analysis of histamine. Anal Biochem 57:388-394, 1974. Hans J Maasch and David G. Marsh. Standardized extract modified allergens allergoids. Clin rev allergy. 5: 89-106; 1987].

It is an *in vitro* assay for detecting released histamine by enzyme-linked fluoroimmunoassay methods after the allergenic activation of basophils by using a patented fluorometric detection method (Reflab, Denmark). The assay is performed with the leukocytes of a washed heparinized whole blood sample stimulated with native *Phleum pratense* allergen extract and with glutaraldehyde-polymerized *Phleum pratense* allergen extract subjected to tangential flow UF and sterilizing filtration (Examples 2 and 3) on ELISA plates. The released histamine is absorbed by a glass fiber matrix inside the well (high affinity and selectivity) and subsequently detected fluorometrically.

The results show the variation in the recognition of both extracts and therefore their allergenicity. The reduction of histamine release at the same protein concentration induced by the *Phleum pratense* allergoid with respect to the non-modified (native) allergen extract can vary from patient to patient, presumably due to the fact that each patient recognizes different allergenic determinants [Hans J Maasch and David G. Marsh. Standardized extract modified allergens allergoids. Clin rev allergy. 5: 89-106; 1987].

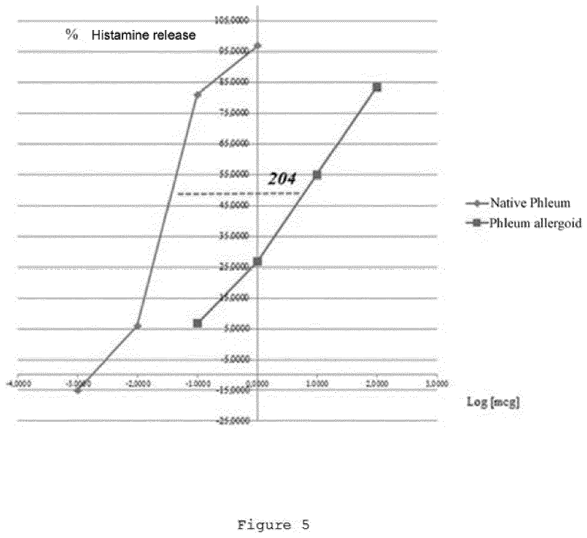

The inventors established a value greater than at least 50, preferably 200 times in the histamine release for native *Phleum pratense* allergen extract compared to glutaraldehyde-polymerized *Phleum pratense* extract subjected to tangential flow UF and sterilizing filtration, which is consistent with what other authors have indicated in the literature [Hans J Maasch & David G. Marsh, Standardized extract modified allergens allergoids. Clin Rev Allergy. (1987). 5: 89-106] (Figure 5).

However, the degree of reduction of the allergenicity in such tests can vary between individuals.

EXAMPLE 6

Safety and efficacy of the method

Intradermal (ID) test is a common method for diagnosing the etiological agent involved in the allergic disease. Once the etiological diagnosis (causal allergen) is established, the modification of its structure with glutaraldehyde allows maintaining its immunogenic character and reducing its allergenicity (ability to induce clinical symptoms). Example 4 illustrates maintaining immunogenicity in the glutaraldehyde-polymerized *Phleum pratense* allergen extract subjected to tangential flow UF and sterilizing filtration as the titers of the specific antibodies obtained in rabbits and the reduction of allergenicity are demonstrated by means of IgE inhibition, allergenic potency and allergen specific histamine release tests shown in Example 5.

The method of using the ID route for therapeutic purposes can be established on these concepts in terms of safety, knowing the concentration of the polymerized allergoid extract that makes the ID skin test negative, in terms of papule or erythema area that is common for the correct interpretation in ID diagnosis.

Concerning efficacy, the ID route has shown to be a route commonly used in immunization against infectious agents [e.g., flu, rabies, hepatitis A and B, influenza, mumps, tetanus, yellow fever, diphtheria-tetanus-pertussis (PATH report-Intradermal Delivery of Vaccines. A review of the literature and the potential for development for use in low- and middle-income countries. August 27, 2009)].

In the specific case of immunization with allergens, the usual treatment concentration range in allergen immunotherapy (IT) corresponds to the concentration proposed by the WHO and described in Examples 2 and 3 and ratified in terms of immunogenicity in Example 4 and according to the allergen values of the WHO report on the use of allergen IT [Allergy 44 (53), 2-42, 1998].

This data supports that the concepts of "maximum tolerated dose" and "optimal dose" can be established by means of ID administration, which means that the administration of allergens in IT can be established in a safe manner, with the absence of side effects, and in an effective manner since the concentration range to be used per each allergen can be previously established by means of the ID skin test with the allergoid.

The safety of the optimal dose can be previously established by means of an end-point titration study with the polymerized allergen extract in a population of patients sensitized to the causal allergen with the polymerized allergen.

Therefore, the concept of maximum effective dose is established by the concentration of the polymerized extract, according to the WHO report by using a polymerized extract that allows increasing the concentrations and adjusting the proposed effective doses due to its allergenicity reducing characteristics demonstrated in Examples 4 and 5.

6.1 Clinical trial

For the main purpose of obtaining the non-reactive maximum dose of *Phleum pratense* allergoid (glutaraldehyde-polymerized *Phleum pratense* allergen), a single-center, open-label, native extract-controlled clinical trial was conducted to obtain the non-reactive maximum dose (end-point titer), with the polymerized *Phleum pratense* allergoid, obtained and characterized as discussed in Examples 1-5, in patients with allergic rhinoconjunctivitis with or without intermittent mild or moderate asthma, sensitized to *Phleum pratense* grass pollen. The safety of said *Phleum pratense* allergoid administered by intradermal route was evaluated as a secondary objective of said clinical trial. The diagnostic variables, including molecular diagnosis, of allergy to *Phleum pratense* were also evaluated.

Number of Patients: 29 patients were recruited. The primary objective of the study was analyzed with the data of 20 patients.

Diagnosis and main inclusion criteria:

1. Patients between 18 and 50 years of age (both inclusive).
2. Evidence of sensitivity to the allergen source:

- Presence of *Phleum pretense-specific* IgE greater than or equal to ($\geq$) 3.5 KU/L (CAP)
- Positive allergen-specific (*Phleum pratense*) skin tests, at least with a papule greater than or equal to ($\geq$) 3 mm with respect to the negative control.

3. Clinical evidence of pathology consistent with clinical symptoms of rhinitis or rhinoconjunctivitis with or without associated intermittent mild or moderate asthma.
4. Patient capable of complying with treatment.
5. Women of child-bearing age must present a negative urine pregnancy test 7 days prior to inclusion in the assay.
6. Patients that meet the preceding criteria and that the investigator considers to have remained stable with respect to their allergic disease for the 6 months prior to entering the study.

All the patients in the trial gave their informed consent to participate in the study.

Drugs and controls used in the trial:

- Experimental drug: Polymerized and lyophilized *Phleum pratense* allergoid, obtained and characterized according to Examples 1-5. Dose: 5, 1, 0.2 and 0.04 $\mu$g of protein/ml. Intradermal route. Batch: 110603
- Positive control: Native *Phleum pratense* extract. Dose: 0.5 ng/ml. Intradermal route. Batch: 120912A.
- Negative control: Mannitol in physiological saline solution. Dose: 10 mg/ml of mannitol with 0.9% saline solution. Intradermal route. Batch: 120912A.

Treatment duration: 1 day of treatment + 1 month of follow-up.

Methodology of the study:

After reconstitution of the drug under study and the controls, they were injected by intradermal route (0.1 mL) in the volar surface of the forearm. The patients received 6 administrations in total: 4 for the experimental drug at different concentrations (5, 1, 0.2 and 0.04 $\mu$g of protein/mL), 1 for the positive control and 1 for the negative control. The produced reaction (papule) around the injection points was measured after 15 minutes. The "non-reactive dose" was considered to be the dose that caused growth of the papule diameter of less than 3 mm without the presence of erythema.

Evaluation criteria:

Primary variable:

Measuring the largest papule diameter. The non-reactive dose is considered that dose which causes a difference in the largest papule diameter of less than 3 mm. To obtain the difference in diameter, the papule is measured immediately after administration of the treatment (initial papule diameter) and measured for a second time at 15 minutes (final papule diameter).

Secondary variables:

- Measuring the largest erythema diameter at 15 minutes and searching for equivalence with the papule diameter observed for each patient.
- Assessing safety by means of documenting and following up on the local and/or systemic adverse events that may be generated in the course of the trial.
- Assessing molecular diagnosis variables in blood samples (total specific IgE, Phl p 1 and 5-specific IgE, IgG, IgG4).

Statistical methods:

Primary variable

Logarithm base 5 of the growth of the largest papule diameter was established for each of the four concentrations used in the study in each of the patients, and a least-squares regression line was calculated with the log values of the papule diameter with respect to the logarithm base 5 of the concentration of the applied doses. The log values of the concentration were interpolated with respect to the log base 5 value equivalent to an increase in the largest papule diameter of 2.9 mm, which is the highest value considered negative in the skin test. The fifth power was applied to the value obtained from the interpolation to obtain a concentration value equivalent to an increase of the largest papule diameter of 2.9 mm. The concentration data was ordered from lowest to highest and the median was calculated.

Secondary variables:

- Measuring the sum of the largest erythema diameter at 15 minutes and searching for equivalence with the papule diameter observed for each patient.
Logarithm base 5 of the largest erythema diameter was calculated for each of the four concentrations used in the study in each of the patients. The log values of diameter with respect to the log value base 5 equivalent at a concentration of 0.27 $\mu$g prot/mL of polymerized Phl p were interpolated on logarithm base 5, said value being the highest value that is considered negative in the skin test, assessing growth of the largest papule diameter. The fifth power was applied to the value obtained from the interpolation to obtain a value of the largest erythema diameter which is considered negative in the skin test.
- Evaluating safety: Descriptive analysis of the local and systemic adverse events, classified by severity and frequency.
- Quantitatively analyzing the variation in the levels of total specific IgE, Phl p 1 and 5-specific IgE, IgG, IgG4 after treatment with respect to their corresponding baseline levels by means of the ImmunoCAP® commercial method.

Results:

Primary variable: A value of 0.27 $\mu$g prot/mL was obtained as the concentration of polymerized *Phleum pratense* that is considered negative in the skin test.
Secondary variable:

- The largest erythema diameter that is considered negative in the skin test was established at 7 mm.
- There are no statistically significant changes in the variation in the levels of total specific IgE, Phl p 1 and 5-specific IgE, IgG and IgG4.
- No adverse events occurred.

Conclusion:

The value of the non-reactive maximum dose of polymerized *Phleum pratense* administered by intradermal route, objective of the study, was established at 0.27 $\mu$g prot/ml. Furthermore, the largest erythema diameter that is considered negative in the skin test was established at 7 mm.
The high tolerability of treatment and the absence of adverse events indicate that the intradermal injection of polymerized *Phleum pratense* is a safe administration route for patients. That is important for developing immunotherapy treatment with the polymerized *Phleum pratense* allergoid.
The evaluation of the diagnosis variables, including molecular diagnosis, of an allergy to *Phleum pratense* does not show statistically significant variations (p$\leq$0.05) in final immunoglobulin levels with respect to initial immunoglobulin levels.

EXAMPLE 7

Studies of the reduction of potency between native and glutaraldehyde-polymerized allergen for *Parietaria judaica* and *Artemisia vulgaris*

7.1 Materials

The *Parietaria judaica* and *Artemisia vulgaris* pollen raw material was processed as described in Example 1, section 1.1, relating to obtaining a pollen allergen extract of the patent for obtaining native allergen extracts.
Then the native allergen extract was characterized as described in point 1.2, section 1.2.1 "Polyacrylamide gel electrophoresis" in Example 1.

The native *Parietaria judaica* and *Artemisia vulgaris* extracts were polymerized with glutaraldehyde in a manner similar to that described for *Phleum pratense.* Briefly, the grams of lyophilized allergen extract necessary to obtain a concentration comprised between 0.1 and 3 mg/mL of protein are weighed in a Eurotubo® sterile IVD, using phosphate buffer saline (PBS), pH 7.4, as a diluent. Then glutaraldehyde is added drop-wise until obtaining a concentration of between 0.01 M and 0.3 M, with gentle stirring (80 rpm) in a magnetic stirrer in a cold chamber at a temperature comprised between 2°C and 8°C for 24 hours, whereby obtaining the corresponding crude polymerized allergen extracts.

The crude polymerized allergen extracts are subjected to tangential flow UF (Labscale® Millipore) through a 300 kDa size exclusion cartridge (Pellicon® XL Filter Millipore), 10 volumes of injection-grade water with respect to the volume of crude polymerized allergen extract being passed through. The resulting polymerized allergen extracts contain the polymerized proteins with a molecular weight greater than 100,000 Da.

7.2 Methods

7.2.1 Polyacrylamide gel electrophoresis

Buffers and solutions

Acrylamide/bisacrylamide, 1.5 M Tris-HCl, pH 8.8; 0.5 M

Tris-HCl, pH 6.8 (Stacking); 10% sodium dodecyl sulfate (SDS) solution; 30 mg/mL ammonium persulfate; Temed (N,N,N',N'-tetramethylethylenediamine); 5X sample buffer; electrode buffer, pH 8.3; molecular weight standard; Coomassie (staining) solution; Coomassie destaining solution.

Method

The gels used are prepared with the following proportions of acrylamide: separating gel 12.5% and stacking gel 4%.

15 $\mu$g protein extract/lane are loaded for Coomassie staining (10 $\mu$L of a sample diluted to 1.5 mg protein extract/mL are loaded)

Once the samples are diluted to the protein concentration necessary for staining, the samples are prepared; to that end, 80 $\mu$L of the previously obtained dilution and 20 $\mu$L of 5X sample buffer (4:1 ratio) are taken in an Eppendorf tube, and the mixture is heated for 8 minutes at about 100°C. After cooling (room temperature), the insoluble residues are removed by centrifuging for 5 minutes at 14,000 rpm.

Electrophoretic conditions

The following are dispensed:

- in one lane, 7 $\mu$L of molecular weight standard for Coomassie staining,
- in one lane, 10 $\mu$L of IHR (in-house reference), and
- in three lanes, 10 $\mu$L of polymerized allergen extract to be analyzed.

When the migration front is close to the end of the gel, at about 45 minutes after having started electrophoresis, the source is disconnected and the gel is removed. The gel is subsequently stained and destained depending on the protein concentration each sample has:

- Coomassie staining: The gel is placed on a 10 cm x 10 cm plate and the Coomassie solution is added. Staining: 1 hour at 60°C, 2 hours at 37°C, or 12 hours at room temperature.
- Coomassie destaining solution: The Coomassie destaining solution is added until the bottom of the gel is almost transparent and the bands of the standard and the samples can be seen well. Once it is completely decolorized, the solution is disposed of and water is added so that it is in optimal hydration conditions.

The molecular weights are determined by the Weber and Osborn method [Weber, K. & Osborn, M. J. Biol. Chem. 244:4406-4412 (1969)], by constructing a calibration line, representing the mobility (in mm) manifested by a series of proteins with respect to the logarithm of their molecular weight (known). Mobility is measured from the start of the separating gel to the midpoint of the band. Once the correlation between both variables ($r > 0.90$) is demonstrated, the molecular weights are determined with a computer program (Diversity database) based on the Weber and Osborn method. The molecular weights of the proteins used as standards are: 250, 150, 100, 75, 50, 37, 25, 20.15 and 10 kDa.

7.2.2 <u>ELISA inhibition with human serum</u>

The products to be analyzed (about 5-6 mg) are weighed on a scale. The weighed samples are reconstituted at 10 mg/mL with pure water, shaken and centrifuged for 5 minutes at 14,000 rpm.

A solution of the reference extract (IHR) to be analyzed is prepared in 50 mM carbonate/bicarbonate buffer, pH 9.6 at a concentration of 250 $\mu$g/mL (6 mL of carbonate/bicarbonate buffer and 150 $\mu$L of IHR at a concentration of 10 mg/mL to be analyzed).

50 $\mu$L of IHR solution (250 $\mu$g/mL) are pipetted into the 96 wells of the ELISA plate and the plate is covered to prevent evaporation. It is incubated overnight at a temperature comprised between 2°C and 8°C in a refrigerator.

The ELISA plate is washed with 0.05% PBS-Tween® 20, pH 7.4, in a Biotek Elx50 washer. It is then blocked by adding 200 $\mu$L/well of 1% BSA in 0.05% PBS-Tween® 20 and incubated at room temperature for 1 hour.

Preparation of the samples to be analyzed. The allergen extracts to be analyzed are prepared at a concentration 5 times greater than the concentration of the IHR for the first point of the line. Half dilutions will be performed in 1% BSA in 0.05% PBS-Tween® 20 from that first point.

To prepare the positive control (BSA + human serum) 90 $\mu$L of 1% BSA in 0.05% PBS-Tween® 20 (instead of the sample) plus 90 $\mu$L of the corresponding human serum are added to an Eppendorf tube.

To prepare the negative control (BSA), 180 $\mu$L of 1% BSA in 0.05% PBS-Tween® 20 are added to an Eppendorf tube.

The samples are incubated together with the positive control and the negative control for 1 hour at room temperature and while being rotated. After the incubation time of the samples has elapsed, they are centrifuged for 10 seconds at 14,000 rpm. 50 $\mu$L of the samples are pipetted into an ELISA plate and the plate is incubated for 2 hours at room temperature. After the blocking time (2 hours) has elapsed, the ELISA plate is washed with 0.05% PBS-Tween® 20, pH 7.4. 50 $\mu$L/well of anti-IgE primary antibody at a dilution of 1/1000 are added and it is incubated for 30 minutes at room temperature. The plate is washed with 0.05% PBS-Tween® 20, pH 7.4. Then, 50 $\mu$L/well of anti-IgG secondary antibody at a dilution of 1/500 are added and it is incubated for 30 minutes at room temperature. The wells are washed with 0.05% PBS-Tween® 20, pH 7.4. Subsequently, 50 $\mu$L/well of the streptavidin-peroxidase conjugate of *Streptomyces avidinii* at a dilution of 1:250 (24 $\mu$L + 6 mL of 1% BSA in 0.05% PBS-Tween® 20) are added and it is incubated for 30 minutes at room temperature. The wells are washed with 0.05% PBS-Tween® 20, pH 7.4. Next, 50 $\mu$L/well of $H_2O_2$ + ABTS (2,2'-azino-bis(3-ethylbenzothiazoline-6-sulfonic acid) at a dilution of 1:1000 (6 $\mu$L of $H_2O_2$ + 6mL ABTS) are added. The plate is read using a Multiskan Ascent V1.23 plate reader.

The Ag 50 for each tested allergoid is calculated:

<u>Log Ag50 and Ag50 value</u>: These correspond with the mean allergen concentration that produces 50% inhibition of IgE binding to the plate.

The LogAg50 value is obtained by extrapolating the different percentages of inhibition with respect to the different allergen concentration logarithms to calculate the allergen concentration capable of causing a 50% inhibition.

The Ag50 value is obtained by calculating the antilogarithm of the LogAg50 value. This value serves to compare allergen extracts to one another, as well as a preparation, treatment or diagnosis with respect to their IHR.

Acceptance limits: The acceptance limits for the allergoids are established at a loss of potency, calculated by comparison of Ag50, with respect to the IHR of more than 90%.

7.2.3 <u>Semi-dry immunoblotting method</u>

Electrophoresis performed according to the conditions described in the "Polyacrylamide gel electrophoresis" method explained above is used as a basis to perform this method.

The PVDF membrane and the paper filter are then cut to the dimensions of the gel, and the paper filter is introduced in a cuvette with transfer buffer. Given the hydrophobic nature of the membrane and in order to make it permeable, the following treatment is performed in a cuvette and under gentle stirring for 10 seconds in methanol. The methanol is then discarded in a waste container and it is immersed for 1 minute in pure water. Finally, the water is discarded in a waste container and it is immersed for 15 minutes in transfer buffer, together with the nitrocellulose filters.

The gel receives prior equilibrium treatment by immersing it in transfer buffer for about 1 minute once electrophoresis has ended.

The transfer components are then placed on the plate of the transfer equipment. To that end, the transfer equipment is opened, and the gray bottom plate is moistened with transfer buffer. The lower paper filter moistened with transfer buffer is then placed. Next the PVDF membrane and the gel, in which the loading wells are previously cut, are placed on the membrane. Care must be taken so that there are no bubbles. Finally, the second paper filter, the upper plate and the transfer equipment cover are placed.

The area of the gel in cm$^2$ (side x side) is then calculated, is multiplied by the constant 5.5 and is divided by 1000. This value gives the milliamperes (mA) required for the transfer. The approximate time of the transfer is 45 minutes.

The amperage and time necessary are selected in the power source and the START button is pressed.

Immunodetection of proteins transferred to the membrane

The proteins transferred to the PVDF membrane are detected by the method of Shen *et al.* (1988) with modifications. Once the proteins are transferred, the membrane is cut to separate the various lanes that are going to be individually incubated, depending on the allergen that is going to be detected. The lower left corner of each of the segments is cut to know the orientation of the membrane.

The membrane is incubated for 1 hour under gentle and constant agitation at room temperature with 0.5% PBS-Tween buffer to saturate the membrane (membrane blocking) and thus prevent subsequent non-specific binding of both immunoglobulins and immunoglobulin conjugates.

To remove the residues of the aforementioned saturation buffer, the membrane is washed with 0.05% PBS-Tween buffer. Five washings lasting 5 minutes each are performed at room temperature and under gentle and constant agitation.

The membrane is then incubated with the corresponding serum diluted in 0.5% PBS-Tween at 4°C and under constant agitation overnight (about 16 hours) in a previously sealed plastic bag.

After incubating with serum, the non-fixed antibodies are removed by means of 5 washings lasting 5 minutes with 0.05% PBS-Tween buffer at room temperature and under gentle and constant agitation.

The membrane is incubated with mouse anti-human IgE antibody, which is diluted to a dilution of 1/1000 in 0.5% PBS-Tween buffer. Then a sufficient volume for covering the membrane is added to it. It is incubated for 2 hours at room temperature under constant agitation.

To remove the non-fixed conjugate, 5 washings lasting 5 minutes with 0.05% PBS-Tween buffer are performed at room temperature and under gentle and constant agitation.

Developing the immunocomplex using PerkinElmer reagents

The developing reagents are prepared: developer/replenisher, dilution of 1/5 (160 mL $H_2O_2$ and 40 mL reagent) in cuvette no. 1. Pure water in cuvette no. 2. Fixer dilution of 1/4 (150 mL $H_2O_2$ and 50 mL Reagent) in cuvette no. 3 and pure water in cuvette no. 4.

Next the enhanced luminol reagent (A) and oxidizing reagent (B) solutions are mixed immediately before use at a ratio of 1:1 (for example: 1 mL of solution A + 1 mL of solution B = 2 mL, for two allergen lanes). The total volume necessary will depend on of the number of lanes to be developed. The final volume required is 0.1 mL/cm$^2$ of membrane. This reagent is left already prepared for the following step.

The excess washing buffer is then removed from the membrane, and the membrane is placed on glass, plastic or another clean surface with the side having the proteins adsorbed thereon facing upwards. From this moment on, the process must be performed in a dark room with a red safety light.

The reagent prepared in the preceding step is added. The surface of the membrane has to be completely immersed. It is incubated for 5 minutes at room temperature. The excess detection mixture is removed (for example, holding the membrane in the vertical position and then letting the liquid run down, touching the lower end of the membrane with drying paper to remove the liquid accumulated on the lower edge).

The membrane is placed on plastic, with the side having the proteins adsorbed thereon facing upwards. The membrane is wrapped in transparent plastic and the air bubbles trapped between the plastic and the membrane are carefully and gently removed, not applying pressure on the membrane. The membrane wrapped in plastic is placed inside the developing cassette with the side having the proteins absorbed thereon facing upwards, making certain that the cassette is dry (there cannot be any reagent residues wetting the cassette) and the film cannot be wet either.

Developing film is placed on the membrane and the plastic. The cassette is closed and exposed for 1 minute. The exposed film is removed with tweezers and gloves and another non-exposed film is placed directly on the membrane.

The first film is developed immediately, passing it through each of the 4 previously prepared reagents for 10 seconds (it will have to be exposed in the developer/replenisher until black spots appear corresponding with the bands fixed by IgE binding to the membrane).

The amount of time for which exposure must be maintained in the second film will be estimated depending on the result in the first film. This exposure can vary between 1 minute and 1 hour depending on the intensity with which the bands are shown.

7.3 Results

7.3.1 Biochemical characterization and ELISA inhibition of a glutaraldehyde-polymerized *Parietaria judaica* pollen allergen extract *vs.* a native *Parietaria judaica* pollen allergen extract

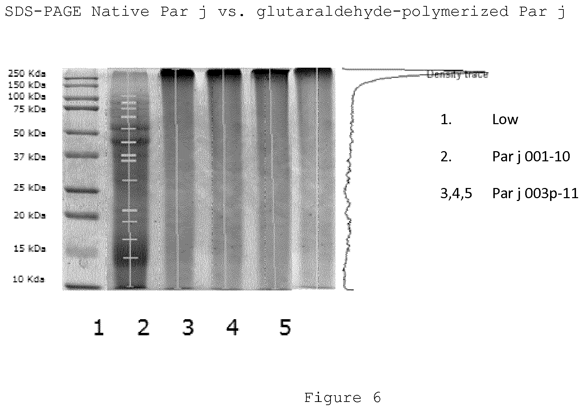

Figure 6 shows the comparison between the native extract (Par j 001-10, lane 2) and the glutaraldehyde-polymerized extract (Par j 003p-11, lanes 3, 4 and 5) by means of polyacrylamide gel electrophoresis and Coomassie blue staining. Said figure shows how the polymerized allergen has no electrophoretic bands below 100 kDa, unlike the native allergen which shows electrophoretic bands at different levels.

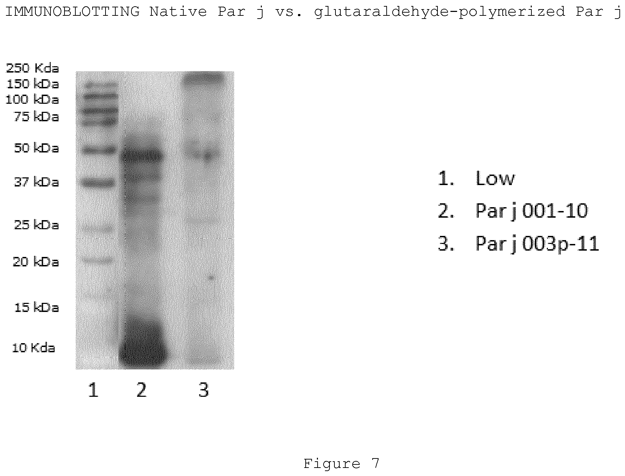

Figure 7 shows the comparison between the native extract (Par j 001-10, lane 2) and the glutaraldehyde-polymerized extract (Par j 003p-11, lane 3) by means of immunoblotting. The figure shows how the polymerized allergen shows no specific IgE fixing below 100 kDa, unlike the native allergen which shows specific IgE fixing at different levels.

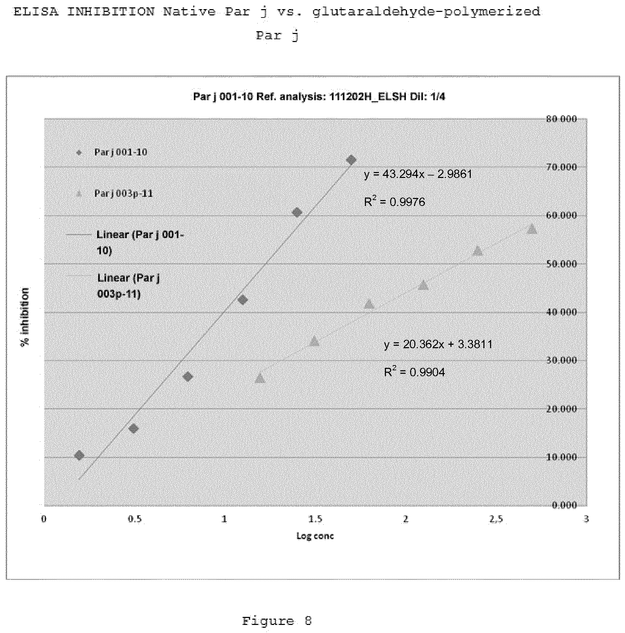

Figure 8 shows the comparison between the native extract (Par j 001-10) and the glutaraldehyde-polymerized extract (Par j 003p-11) by means of ELISA inhibition, showing a loss of potency of 91.46%.

7.3.2 Biochemical characterization and ELISA inhibition of a glutaraldehyde-polymerized *Artemisia vulgaris* pollen allergen extract *vs.* a native *Artemisia vulgaris* pollen allergen extract

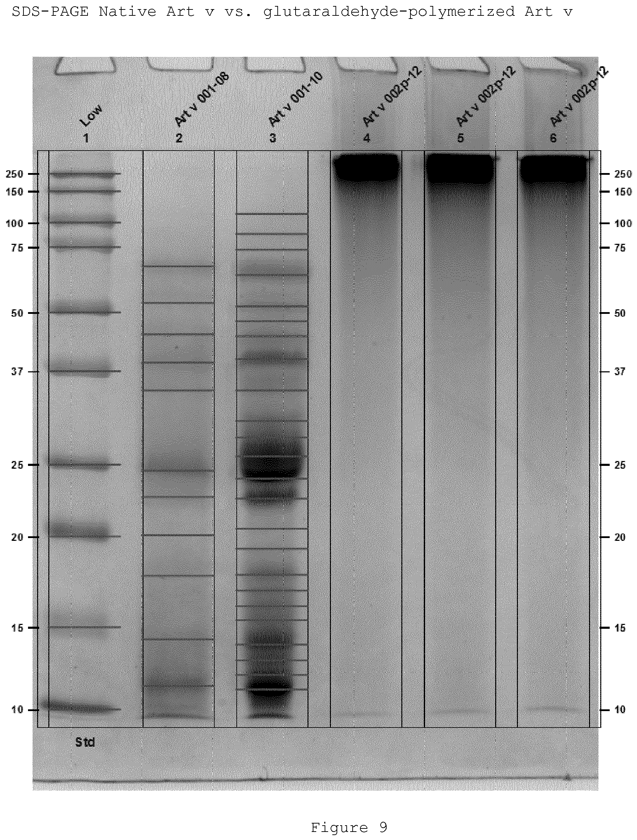

Figure 9 shows the comparison between the native extract (Art v 001-06 and Art v 001-10, lanes 2 and 3) and the glutaraldehyde-polymerized extract (Art v 002p-12, lanes 4, 5 and 6) by means of polyacrylamide gel electrophoresis and Coomassie blue staining. The figure shows how the polymerized allergen has no electrophoretic bands below 100 kDa, unlike the native allergens which show electrophoretic bands at different levels.

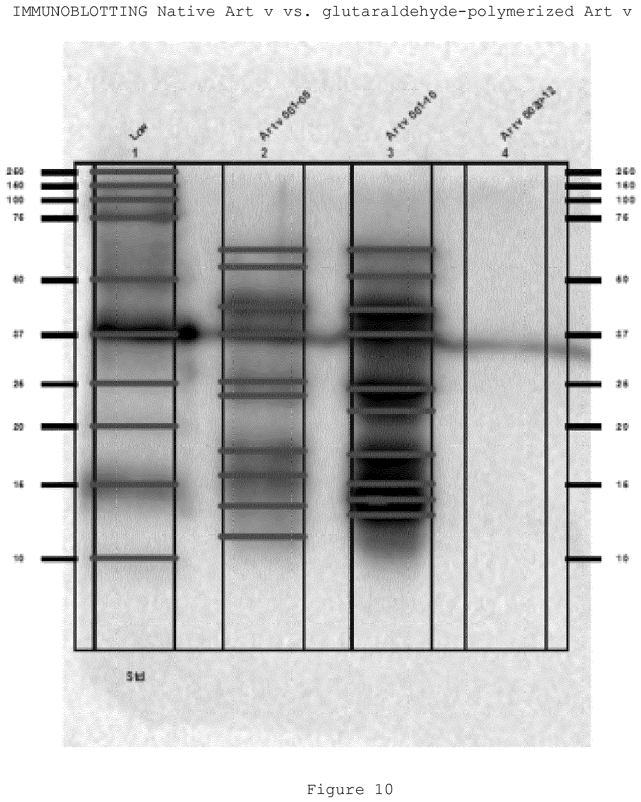

Figure 10 shows the comparison between the native extract (Art v 001-06 and Art v 001-10, lanes 2 and 3) and the glutaraldehyde-polymerized extract (Art v 002p-12, lane 4) by means of immunoblotting. The figure shows how the polymerized allergen shows no specific IgE fixing below 100 kDa, unlike the native allergens which show specific IgE fixing at different levels.

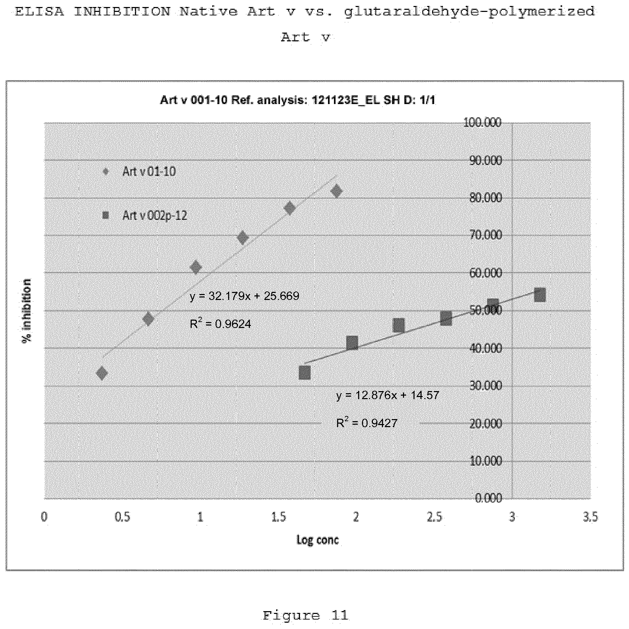

Figure 11 shows the comparison between the native extract (Art v 001-10) and the glutaraldehyde-polymerized extract (Art v 002p-12) by means of ELISA inhibition, showing a loss of potency of 98.91%.

7.4 Discussion

The process of polymerization with glutaraldehyde shows the absence of specific IgE fixing bands in the molecular weight (MW) ranges of the allergens described by means of immunofixing and ELISA inhibition techniques showing the reduction of the IgE fixing capacity of glutaraldehyde-polymerized extracts vs. native extracts in terms of relative potency (RP), showing a loss of relative potency of 91.4% for the glutaraldehyde-polymerized *Parietaria judaica* allergen extract and of 98.9% for the glutaraldehyde-polymerized *Artemisia vulgaris* allergen extract.

EXAMPLE 8

Obtaining the vials for treatment with glutaraldehyde-polymerized *Dermatophagoides pteronyssinus* allergen extract

About 0.3 g lyophilized *Dermatophagoides pteronyssinus* allergen extract (250 mg protein/g of extract) comprising at least Der p 1, Der p 2, Der p 3, Der p 6 and Der p 10 are weighed in a Eurotubo® sterile IVD. This extract is from in-house cultures grown and controlled in the laboratory. The extract is diluted in PBS, pH 7.4, at a concentration comprised between 0.1 and 3 mg/mL of protein to obtain a volume of 50 mL. Between 0.1 and 2 mL, typically 0.5 mL, of glutaraldehyde are then added drop-wise to a concentration between 0.01 M and 0.3 M, typically 0.025 M, with gentle stirring (80 rpm) in a magnetic stirrer in a cold chamber at a temperature comprised between 2°C and 8°C for 24 hours, whereby obtaining a crude polymerized *Dermatophagoides pteronyssinus* allergen extract.

Said crude polymerized allergen extract is subjected to tangential flow UF (Labscale® Millipore) through a 300 KDa size exclusion cartridge (Pellicon® XL Filter Millipore), 10 volumes of injection-grade water with respect to the volume of crude polymerized allergen extract, between 500 mL and 1,500 mL, being passed through. The resulting glutaraldehyde-polymerized *Dermatophagoides pteronyssinus* allergen extract contains the polymerized proteins with a molecular weight greater than 100,000 Da. The protein concentration present in the native allergen starting extract can be determined as indicated in Example 1.

Once the glutaraldehyde-polymerized *Dermatophagoides pteronyssinus* allergen extract subjected to tangential flow UF, obtained as described above, is dialyzed in a Labscale tangential flow UF system, it is subjected to sterilizing filtration and the concentration of the polymerized allergen extract is adjusted as described below.

To carry out the sterilizing filtration of said polymerized allergen extract subjected to tangential flow UF, the

0.22 micrometer ($\mu$m) polyethersulfone sterilizing filter is previously activated. 2 mL of mannitol (5 mg/mL) are added to the filter and it is left to incubate for 5 minutes; then the vacuum system valve is opened until all the buffer is filtered (mannitol in water for injection at 5 mg/mL), and the vacuum system valve is subsequently closed. This process is repeated once more. Then the filter is left to dry for 30 minutes (15 minutes with an open vacuum and another 15 minutes with a closed vacuum), 2.5 mL of the sample to be filtered (polymerized allergen extract subjected to tangential flow UF) are added and the vacuum valve is opened for 10 minutes until the filter dries, this step being repeated 2 more times. The filter is finally left to dry for 10 minutes.

Once the 0.22 $\mu$m polyethersulfone sterilizing filter is activated, the polymerized allergen extract subjected to tangential flow UF is subjected to sterilizing filtration. To that end, before filtering said polymerized allergen extract subjected to tangential flow UF, a 2 mL sample is taken to analyze the biological load of the product. Then the vacuum is opened and the polymerized allergen extract subjected to tangential flow UF is added to be filtered and once filtered, it is passed to a sterile chamber through the SAS system [Security Air System]. A 10 mL aliquot of said polymerized allergen extract subjected to tangential flow UF and sterilizing filtration is analyzed by means of the Bradford assay [Bradford, M.M. (1976), Anal. Biochem. 72: 248-254] to determine the amount of protein present in said extract and to subsequently perform the bulk packaging. The polymerized allergen extract subjected to tangential flow UF and sterilizing filtration is diluted in cryoprotective diluent (mannitol at 10 mg/mL) to obtain a final concentration of between 0.001 and 1,000 $\mu$g of protein per mL, typically at 0.02 mg of protein/mL in the polymerized allergen extract subjected to tangential flow UF and sterilizing filtration. After adjusting to protein treatment concentration the obtained bulk product is metered into 3 mL capacity vials at a proportion of 1 mL/vial. Once the vials are metered, they are lyophilized by introducing them in a Telstar lyophilizer and performing a complete lyophilization cycle, which ends by vacuum sealing of the vials.

EXAMPLE 9

Biochemical characterization and ELISA inhibition of a glutaraldehyde-polymerized *Dermatophagoides pteronyssinus* allergen extract, final product for intradermal administration

The biochemical characterization and ELISA inhibition of the *D. pteronyssinus* allergoid obtained in Example 8 were performed.

9.1 Methods of biochemical characterization and ELISA inhibition 9.1.1 Polyacrylamide gel electrophoresis

The protocol described in Example 7, section 7.2.1, was followed. Native *Dermatophagoides pteronyssinus* extract was used as an in-house reference (IHR).

9.1.2 ELISA inhibition with human serum

The protocol described in Example 7, section 7.2.2, was followed. The solution of the reference extract (native *Dermatophagoides pteronyssinus* (IHR) extract) to be analyzed was prepared in 50 mM carbonate/bicarbonate buffer, pH 9.6, at a concentration of 250 $\mu$g/mL (6 mL of carbonate/bicarbonate buffer and 150 $\mu$L of IHR at a concentration of 10 mg/mL).

9.1.3 Semi-dry immunoblotting method

The protocol described in Example 7, section 7.2.3, was followed, including that relating to the immunodetection of the proteins transferred to the membrane and the development of the immunocomplex.

9.2 Results

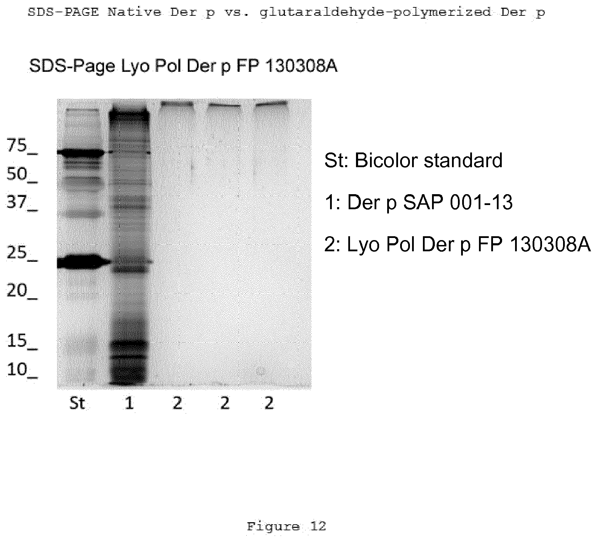

Figure 12 shows the comparison between the native extract (Der p SAP 001-13, lane 1) and the glutaraldehyde-polymerized extract (lyophilized polymerized Der p, lane 2) by means of polyacrylamide gel electrophoresis and Coomassie blue staining. Said figure shows how the electrophoretic bands corresponding to the characteristic *Dermatophagoides pteronyssinus* allergens appear in the native extract, whereas no electrophoretic band below 100 kDa appears in the polymerized allergen.

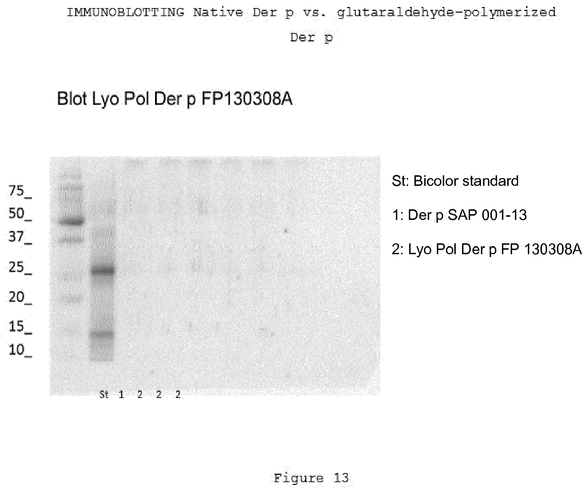

Figure 13 shows the comparison between the native extract (Der p 001-13, lane 1) and the glutaraldehyde-polymerized extract (lyophilized polymerized Der p, lane 2) by means of immunoblotting. Said figure shows how specific IgE fixing bands corresponding to the characteristic *Dermatophagoides pteronyssinus* allergens appear in the native extract, whereas specific IgE fixing below 100 kDa is not detected in the polymerized allergen.

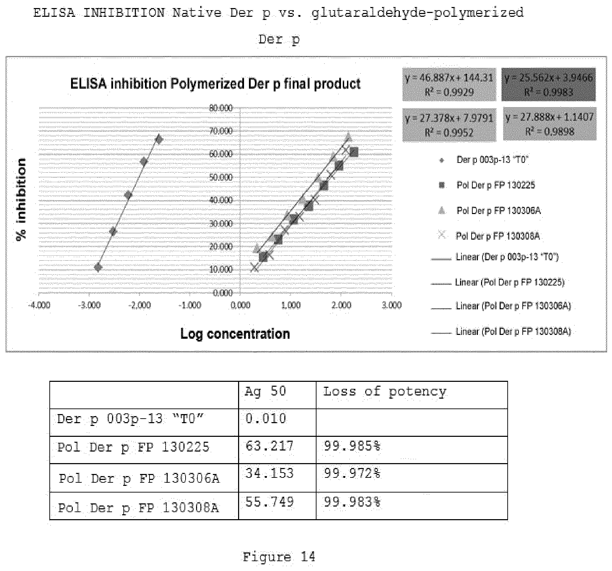

Figure 14 shows the comparison between the native extract (Der p 001-13) and three consecutive batches of lyophilized glutaraldehyde-polymerized Der p extract by means of ELISA inhibition, showing a loss of potency of over 99% in the three cases.

EXAMPLE 10

Obtaining vials for treatment with glutaraldehyde-polymerized *Apis mellifera* venom allergen extract

Between 0.3 and 0.4 g of lyophilized *Apis mellifera* venom raw material (200-250 mg protein/g of extract) comprising at least Api m1, Api m 2 and Api m 3 are weighed in a Eurotubo® sterile IVD. This raw material is acquired from an audited and approved supplier (Allergon, Greer, USA). The raw material is diluted in phosphate buffer saline (PBS), pH 7.4, at a concentration between 0.1 and 3 mg/mL of protein to obtain a volume of 50 mL. Between 0.1 and 2 mL, typically 0.5 mL, of glutaraldehyde are then added drop-wise to a concentration between 0.01 M and 0.3 M, typically 0.025 M, with gentle stirring (80 rpm) in a magnetic stirrer in a cold chamber at a temperature comprised between 2°C and 8°C for 24 hours, whereby obtaining a crude polymerized allergen extract.

Said crude polymerized allergen extract is subjected to tangential flow ultrafiltration (UF) (Labscale® Millipore) through a 300 KDa size exclusion cartridge (Pellicon® XL Filter Millipore), 10 volumes of injection-grade water with respect to the volume of crude polymerized *Apis mellifera* venom allergen extract, between 500 mL and 1,500 mL, being passed through. The resulting polymerized *Apis mellifera* venom allergen extract contains the polymerized proteins with a molecular weight greater than 100,000 Da. The protein concentration present in the starting raw material can be determined as indicated in Example 1.

Once the glutaraldehyde-polymerized *Apis mellifera* venom allergen extract subjected to tangential flow UF, obtained as described above, is dialyzed in a Labscale tangential flow ultrafiltration system, it is subjected to filtration and the concentration of the polymerized allergen extract is adjusted as described below.

To carry out the sterilizing filtration of said polymerized allergen extract subjected to tangential flow UF, the 0.22 micrometer ($\mu$m) polyethersulfone sterilizing filter is previously activated. 2 mL of mannitol (5 mg/mL) are added to the filter and it is left to incubate for 5 minutes; then the vacuum system valve is opened until all the buffer (mannitol in water for injection at 5 mg/mL) is filtered, and the vacuum system valve is subsequently closed. This process is repeated once more. Then the filter is left to dry for 30 minutes (15 minutes with an open vacuum and another 15 minutes with a closed vacuum), 2.5 mL of the sample to be filtered (polymerized allergen extract subjected to tangential flow UF) are added and the vacuum valve is opened for 10 minutes until the filter dries, this step being repeated 2 more times. The filter is finally left to dry for 10 minutes.

Once the 0.22 $\mu$m polyethersulfone sterilizing filter is activated, the polymerized allergen extract subjected to tangential flow UF is subjected to sterilizing filtration. To that end, before filtering said polymerized allergen extract subjected to tangential flow UF, a 2 mL sample is taken to analyze the biological load of the product. Then the vacuum is opened and the polymerized allergen extract subjected to tangential flow UF is added to be filtered and once filtered, it is passed to a sterile chamber through the SAS system [Security Air System]. A 10 mL aliquot of said polymerized allergen extract subjected to tangential flow UF and sterilizing filtration is analyzed by means of the Bradford assay [Bradford, M.M. (1976), Anal. Biochem. 72: 248-254] to determine the amount of protein present in said extract and to subsequently perform the bulk packaging. The polymerized allergen extract subjected to tangential flow UF and sterilizing filtration is diluted in cryoprotective diluent (mannitol at 10 mg/mL) to obtain a final concentration of between 0.001 and 1,000 $\mu$g of protein per mL, typically at 0.01 mg/mL of protein in the polymerized allergen extract subjected to tangential flow UF and sterilizing filtration. After adjusting to protein treatment concentration the obtained bulk product is metered into 3 mL capacity vials at a proportion of 1 mL/vial. Once the vials are metered, they are lyophilized by introducing them in a Telstar lyophilizer and performing a complete lyophilization cycle, which ends by vacuum sealing of the vials.

EXAMPLE 11

Biochemical characterization and ELISA inhibition of glutaraldehyde-polymerized *Apis mellifera* venom, final product for intradermal administration

The biochemical characterization and ELISA inhibition of the allergoid of the *Apis mellifera* venom obtained in Example 10 were performed.

11.1 Methods of biochemical characterization and ELISA inhibition

11.1.1 Polyacrylamide gel electrophoresis

The protocol described in Example 7, section 7.2.1, was followed. Native *Apis mellifera* venom extract was used as an in-house reference (IHR).

### 11.1.2 ELISA inhibition with human serum

The protocol described in Example 7, section 7.2.2, was followed. The solution of the reference extract (native *Apis mellifera* venom (IHR) extract) to be analyzed was prepared in 50 mM carbonate/bicarbonate buffer, pH 9.6, at a concentration of 250 $\mu$g/mL (6 mL of carbonate/bicarbonate buffer and 150 $\mu$L of IHR at a concentration of 10 mg/mL).

### 11.1.3 Semi-dry immunoblotting method

The protocol described in Example 7, section 7.2.3, was followed, including that relating to the immunodetection of the proteins transferred to the membrane and the development of the immunocomplex.

### 11.2 Results

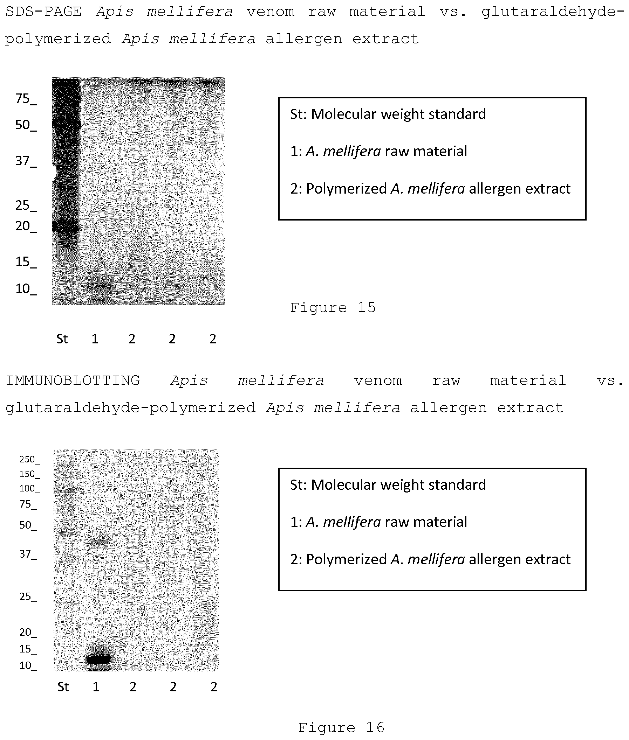

Figure 15 shows the comparison between the *Apis mellifera* venom raw material (lane 1) and the glutaraldehyde-polymerized venom (lane 2) by means of polyacrylamide gel electrophoresis and Coomassie blue staining. Said figure shows how the electrophoretic bands corresponding to the characteristic *Apis mellifera* allergens appear in the raw material, whereas no electrophoretic band below 100 kDa appears in the polymerized allergen.

Figure 16 shows the comparison between the *Apis mellifera* venom raw material (lane 1) and the glutaraldehyde-polymerized venom (lane 2) by means of immunoblotting. Said figure shows how the specific IgE fixing bands corresponding to the characteristic *Apis mellifera* allergens appear in the raw material, whereas specific IgE fixing below 100 kDa is not detected in the polymerized allergen.

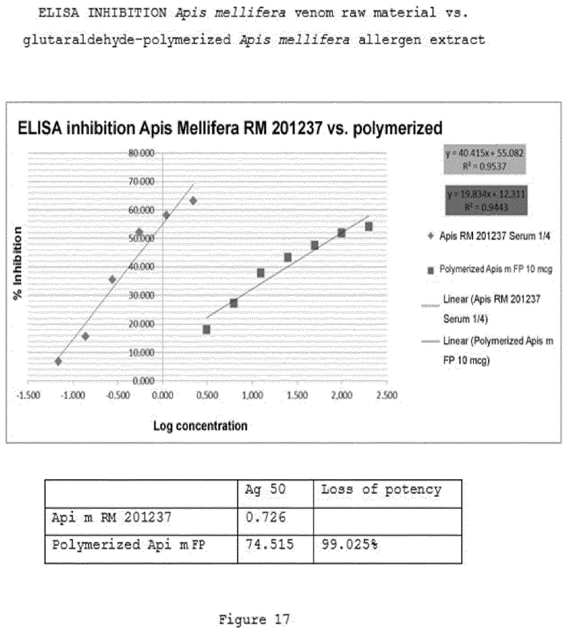

Figure 17 shows the comparison between the *Apis mellifera* venom raw material and the lyophilized glutaraldehyde-polymerized *Apis mellifera* venom allergen extract by means of ELISA inhibition, showing a loss of potency of over 99%.

### EXAMPLE 12

### Healthy donor basophil histamine release assay with the passive addition of IgE from the serum of patients that are allergic to *Apis mellifera* venom

The allergic patient basophil histamine release assay used is an approved method for evaluating the allergenicity of allergenic preparations [Siraganian RP: An automated continuous flow system for the extraction and fluorometric analysis of histamine. Anal Biochem 57:388-394, 1974. Hans J Maasch and David G. Marsh. Standardized extract modified allergens allergoids. Clin rev allergy. 5: 89-106; 1987].

It is an *in vitro* assay for detecting released histamine by enzyme-linked fluoroimmunoassay methods after the allergenic activation of basophils by using a patented fluorometric detection method (Reflab, Denmark). The assay is performed with the basophils of a washed heparinized whole blood sample stimulated with native *Apis mellifera* allergen extract and with glutaraldehyde-polymerized *Apis mellifera* allergen extract (active substance and final product) from one or several atopic patients, or with the basophils (from healthy donors) subjected to the removal of IgEs by a pH reduction and in which histamine contained in these basophils is released by transferring the IgE from the sera of allergic patients (passive transfer) [M. H. Platzer, C. E. H. Grattan, L. K. Poulsen, P. S. Skov. Validation of basophil histamine release against the autologous serum skin test and outcome of serum-induced basophil histamine release studies in a large population of chronic urticaria patients. Allergy 2005: 60: 1152-1156]. The released histamine is absorbed by a glass fiber matrix inside the well (high affinity and selectivity) and subsequently detected fluorometrically.

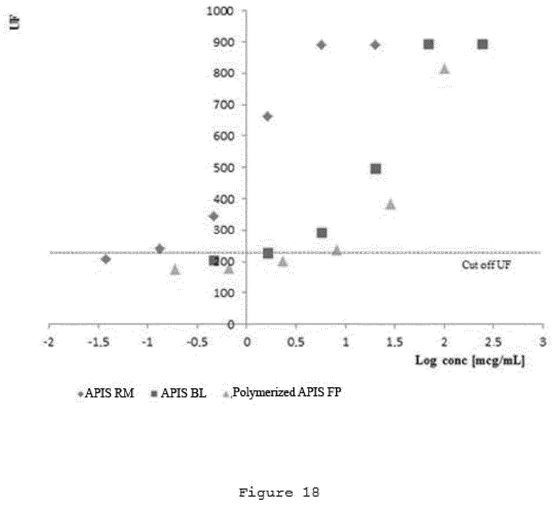

The results show the variation in the recognition of both extracts and therefore their allergenicity. The reduction of histamine release at the same protein concentration induced by the *Apis mellifera* allergoid with respect to the non-modified (native) allergen extract can vary from patient to patient, presumably due to the fact that each patient recognizes different allergenic determinants [Hans J Maasch and David G. Marsh. Standardized extract modified allergens allergoids. Clin rev allergy. 5: 89-106; 1987]. As shown in Figure 18, histamine release at the same concentration of native and polymerized extract (final product) is between 3 and 4 times greater in the case of the native extract, thereby demonstrating the reduction of allergenicity of the polymerized extract with respect to the native extract.

APIS RM (♦) = *Apis mellifera* allergen extract

APIS BL (■) = Crude polymerized *Apis mellifera* extract Polymerized APIS FP (▲) = lyophilized polymerized *Apis mellifera* extract

**Claims**

1.  A pharmaceutical composition for use in the treatment of a subject suffering from an allergy to an allergen, wherein the composition is to be administered intradermally, the composition comprising at least one allergoid and at least one excipient, wherein said allergoid is an allergoid obtained by means of chemically modifying said allergen, wherein said allergoid is present in the composition at the non-reactive maximum dose and wherein said non-reactive maximum dose corresponds to the allergoid concentration that, administered by the intradermal route, makes the skin test negative as tested on a population that is specifically sensitized to said allergen by means of an end-point titration study with the allergoid in a population of patients sensitized to the allergen.

2.  The pharmaceutical composition for use according to claim 1, wherein said allergoid is an allergoid obtained by means of polymerizing an allergen with glutaraldehyde.

3.  The pharmaceutical composition for use according to claim 1 or 2, wherein said allergoid is an allergoid obtained from a plant pollen allergen extract, an allergoid obtained from an animal epidermal derivative allergen extract, an allergoid obtained from a dust mite allergen extract, an allergoid obtained from a fungus allergen extract, an allergoid obtained from a food allergen extract, an allergoid obtained from an animal component allergen extract, or an allergoid obtained from a latex (*Hevea brasiliensis*) allergen extract.

4.  The pharmaceutical composition for use according to any of claims 1 to 3, wherein said allergoid is an allergoid selected from the group consisting of allergoids obtained from an individual allergen or from an allergen extract of Agropyron spp, *Acacia dealbata, Alnus glutinosa, Amaranthus spp, Ambrosia spp, Artemisia vulgaris, Avena sativa, Betula verrucosa, Chenopodium album, Chrysanthemum spp, Citrus sinensis, Corylus avellana, Cryptomeria* japonica, *Cupressus arizonica, Cupressus sempervirens, Cynodon dactylon, Dactylis glomerata, Eucalyptus spp, Fagus sylvatica, Festuca pratensis, Fraxinus excelsior, Helianthus spp, Hevea brasiliensis, Holcus lanatus, Hordeum vulgare, Jasminum spp, Juniperus oxycedrus, Ligustrum vulgare, Lolium perenne, Mercurialis annua, Morus alba, Olea europaea, Oryza sativa, Parietaria judaica, Phleum pratense, Phoenix canariensis, Phoenix dactylifera, Phragmites communis, Phytolacca dioica, Pinus sylvestris, Plantago lanceolata, Platanus acerifolia, Poa pratensis, Populus deltoides, Quercus ilex, Quercus robur, Quercus virginiana, Robinia pseudoacacia, Rumex acetosella, Salix nigra, Salsola kali, Sambucus nigra, Schinopsis sp., Secale cereale, Taraxacum officinale, Trisetum paniceum, Triticum aestivum, Ulmus campestris, Urtica dioica, Zea mays, Canis familiaris, Equus caballus, Felis domesticus, Acarus siro, Blomia kulagini, Blomia tropicalis, Dermatophagoides pteronyssinus, Dermatophagoides farinae, Glycyphagus domesticus, Lepidoglyphus destructor, Tyrophagus putrescentiae, Alternaria alternata, Aspergillus fumigatus, Aspergillus niger, Cladosporium herbarum, Penicillium notatum, Rhizopus nigricans,* fish, milk and milk products, eggs and egg products, nuts and dried fruit, jellyfish venom, snake venom, caterpillar venom, venom of a hymenoptera, venom of a diptera, venom from an insect belonging to the order *Siphonaptera,* latex (*Hevea brasiliensis*), or combinations thereof.

5.  The pharmaceutical composition for use according to any of claims 1 to 4, comprising a single allergoid or two or more allergoids.

6.  The pharmaceutical composition for use according to any of claims 1 to 5, wherein said allergoid is lyophilized and is dissolved in an excipient suitable for administration by intradermal route.

7.  The pharmaceutical composition for use according to any of claims 1 to 6, wherein said excipient comprises physiological saline and/or mannitol.

8.  The pharmaceutical composition for use according to any of claims 1 to 7, in single-dose form.

9.  The pharmaceutical composition for use according to any of claims 1 to 8, wherein the allergoid content per unit dose of 0.1 mL is comprised between about 0.001 $\mu$g of protein and about 1,000 $\mu$g of protein per dose.

10. A pharmaceutical kit comprising a pharmaceutical composition wherein the composition is to be administered intradermally, the composition comprising at least one allergoid and at least one excipient, wherein said allergoid is an allergoid obtained by means of chemically modifying said allergen, wherein said allergoid is present in the composition at the non-reactive maximum dose and wherein said non-reactive maximum dose corresponds to the allergoid concentration that, administered by the intradermal route, makes the skin test negative as tested on a population that is specifically sensitized to said allergen by means of an end-point titration study with the allergoid in a population of patients sensitized to the allergen and the means and instructions for administering said composition for use in the treatment of a subject suffering from an allergy to an allergen.

11. The kit for use according to claim 10, wherein said allergoid is lyophilized and the kit comprises at least one vial with an excipient suitable for administration of the allergoid after reconstitution by intradermal route.

**Patentansprüche**

1. Arzneimittel zur Verwendung bei der Behandlung eines Individuums, das an einer Allergie auf ein Allergen leidet, wobei die Zusammensetzung intradermal zu verabreichen ist, die Zusammensetzung mindestens ein Allergoid und mindestens einen Exzipienten umfasst, wobei das Allergoid ein Allergoid ist, das mittels chemischer Modifizierung des Allergens erhalten wird, wobei das Allergoid in der Zusammensetzung in der nicht-reaktiven Höchstdosis vorliegt und wobei die nicht-reaktive Höchstdosis der Allergoidkonzentration entspricht, die, wenn sie über den intradermalen Verabreichungsweg verabreicht wird, einen negativen Hauttest ergibt, wie an einer spezifisch für das Allergen sensibilisierten Population mittels einer Endpunkt-Titrations-Studie mit dem Allergoid an einer für das Allergen sensibilisierten Patientenpopulation getestet.

2. Arzneimittel zur Verwendung nach Anspruch 1, wobei das Allergoid ein Allergoid ist, das mittels Polymerisierung eines Allergens mit Glutaraldehyd erhalten wird.

3. Arzneimittel zur Verwendung nach Anspruch 1 oder 2, wobei das Allergoid ein Allergoid ist, das aus einem Pflanzenpollen-Allergenextrakt erhalten wird, ein Allergoid, das aus einem tierischen Epidermisderivat-Allergenextrakt erhalten wird, ein Allergoid, das aus einem Hausstaubmilben-Allergenextrakt erhalten wird, ein Allergoid, das aus einem Pilz-Allergenextrakt erhalten wird, ein Allergoid, das aus einem Nahrungsmittel-Allergenextrakt erhalten wird, ein Allergoid, das aus einem Tierkomponenten-Allergenextrakt erhalten wird, oder ein Allergoid, das aus einem Latex (*Hevea brasiliensis*)-Allergenextrakt erhalten wird.

4. Arzneimittel zur Verwendung nach einem der Ansprüche 1 bis 3, wobei das Allergoid ein Allergoid ist, ausgewählt aus der Gruppe bestehend aus Allergoiden, die aus einem Einzelallergen oder aus einem Allergenextrakt aus *Agropyron spp., Acacia dealbata, Alnus glutinosa, Amaranthus spp., Ambrosia spp., Artemisia vulgaris, Avena sativa, Betula verrucosa, Chenopodium album, Chrysanthemum spp., Citrus sinensis, Corylus avellana, Cryptomeria japonica, Cupressus arizonica, Cupressus sempervirens, Cynodon dactylon, Dactylis glomerata, Eucalyptus spp., Fagus sylvatica, Festuca pratensis, Fraxinus excelsior, Helianthus spp., Hevea brasiliensis, Holcus lanatus, Hordeum vulgare, Jasminum spp., Juniperus oxycedrus, Ligustrum vulgare, Lolium perenne, Mercurialis annua, Morus alba, Olea europaea, Oryza sativa, Parietaria judaica, Phleum pratense, Phoenix canariensis, Phonix dactylifera, Phragmites communis, Phytolacca dioica, Pinus sylvestris, Plantago lanceolata, Platanus acerifolia, Poa pratensis, Populus deltoides, Quercus ilex, Quercus robur, Quercus virginiana, Robinia pseudoacacia, Rumex acetosella, Salix nigra, Salsola kali, Sambucus nigra, Schinopsis sp., Secale cereale, Taraxacum officinale, Trisetum paniceum, Triticum aestivum, Ulmus campestris, Urtica dioica, Zea mays, Canis familiaris, Equus caballus, Felis domesticus, Acarus siro, Blomia kulagini, Blomia tropicalis, Dermatophagoides pteronyssinus, Dermatophagoides farinae, Glycyphagus domesticus, Lepidoglyphus destructor, Tyrophagus putrescentiae, Alternaria alternata, Aspergillus fumigatus, Aspergillus niger, Cladosporium herbarum, Penicillium notatum, Rhizopus nigricans,* Fisch, Milch und Milchprodukte, Eier und Eiprodukte, Nüsse und Trockenfrüchte, Quallengift, Schlangengift, Raupengift, Gift eines Hautflüglers, Gift eines Zweiflüglers, Gift von einem Insekt, das zur Ordnung *Siphonaptera* gehört, Latex (*Hevea brasiliensis*) oder Kombinationen davon.

5. Arzneimittel zur Verwendung nach einem der Ansprüche 1 bis 4, das ein Einzelallergoid oder zwei oder mehr Allergoide umfasst.

6. Arzneimittel zur Verwendung nach einem der Ansprüche 1 bis 5, wobei das Allergoid lyophilisiert und in einem zur Verabreichung über den interdermalen Verabreichungsweg geeigneten Exzipienten gelöst ist.

7. Arzneimittel zur Verwendung nach einem der Ansprüche 1 bis 6, wobei der Exzipient physiologische Kochsalzlösung und/oder Mannitol ist.

8. Arzneimittel zur Verwendung nach einem der Ansprüche 1 bis 7 in Form einer Einzeldosis.

9. Arzneimittel zur Verwendung nach einem der Ansprüche 1 bis 8, wobei der Allergoidgehalt pro Dosiseinheit von 0,1 ml zwischen etwa 0,001 μg Protein und etwa 1,000 μg Protein pro Dosis umfasst ist.

10. Arzneimittel-Kit, umfassend ein Arzneimittel, wobei die Zusammensetzung intradermal zu verabreichen ist, die Zusammensetzung mindestens ein Allergoid und mindestens einen Exzipienten umfasst, wobei das Allergoid ein Allergoid ist, das mittels chemischer Modifikation erhalten wird, wobei das Allergoid in der Zusammensetzung in der nicht-reaktiven Höchstdosis vorliegt und wobei die nicht-reaktive Höchstdosis der Allergoid-Konzentration entspricht, die, wenn sie über den intradermalen Verabreichungsweg verabreicht wird, einen negativen Hauttest ergibt, wie an einer spezifisch für das Allergen sensibilisierten Population mittels einer Endpunkt-Titrations-Studie mit dem Allergoid an einer für das Allergen sensibilisierten Patientenpopulation getestet, und Mittel und Anweisungen zur Verabreichung der Zusammensetzung zur Verwendung bei der Behandlung eines Individuums, das an einer Allergie gegen ein Allergen leidet.

11. Kit zur Verwendung nach Anspruch 10, wobei das Allergoid lyophilisiert ist und der Kit mindestens eine Ampulle mit einem Exzipienten, der für die Verabreichung des Allergoids nach Rekonstitution über den intradermalen Verabreichungsweg geeignet ist.

## Revendications

1. Une composition pharmaceutique destinée à être utilisée dans le traitement d'un sujet souffrant d'une allergie à un allergène, la composition étant destinée à être administrée par voie intradermique, la composition comprenant au moins un allergoïde et au moins un excipient, ledit allergoïde étant un allergoïde obtenu au moyen d'une modification chimique dudit allergène, ledit allergoïde étant présent dans la composition à la dose maximale non réactive et ladite dose maximale non réactive correspondant à la concentration d'allergoïde qui, administrée par voie intradermique, rend le test cutané négatif tel que testé sur une population qui est spécifiquement sensibilisée audit allergène au moyen d'une étude finale de titrage avec l'allergoïde chez une population de patients sensibilisés à l'allergène.

2. La composition pharmaceutique destinée à être utilisée selon la revendication 1, dans laquelle ledit allergoïde est un allergoïde obtenu par polymérisation d'un allergène avec du glutaraldéhyde.

3. La composition pharmaceutique destinée à être utilisée selon la revendication 1 ou la revendication 2, dans laquelle ledit allergoïde est un allergoïde obtenu à partir d'un extrait d'allergène de pollen de plante, un allergoïde obtenu à partir d'un extrait d'allergène dérivé d'épiderme d'animal, un allergoïde obtenu à partir d'un extrait d'allergène d'acarien, un allergoïde obtenu à partir d'un extrait d'allergène de champignon, un allergoïde obtenu à partir d'un extrait d'allergène de nourriture, un allergoïde obtenu à partir d'un extrait d'allergène de composant animal, ou un allergoïde obtenu à partir d'un extrait d'allergène de latex (*Hevea brasiliensis*).

4. La composition pharmaceutique destinée à être utilisée selon l'une quelconque des revendications 1 à 3, dans laquelle ledit allergoïde est un allergoïde choisi dans le groupe constitué par les allergoïdes obtenus à partir d'un allergène individuel ou d'un extrait allergénique d'*Agropyron spp, Acacia dealbata, Alnus glutinosa, Amaranthus spp, Ambrosia spp, Artemisia vulgaris, Avena sativa, Betula verrucosa, Chenopodium album, Chrysanthemum spp, Citrus sinensis, Corylus avellana, Cryptomeria japonica, Cupressus arizonica, Cupressus sempervirens, Cynodon dactylon, Dactylis glomerata, Eucalyptus spp, Fagus sylvatica, Festuca pratensis, Fraxinus excelsior, Helianthus spp, Hevea brasiliensis, Holcus lanatus, Hordeum vulgare, Jasminum spp, Juniperus oxycedrus, Ligustrum vulgare, Lolium perenne, Mercurialis annua, Morus alba, Olea europaea, Oryza sativa, Parietaria judaica, Phleum pratense, Phoenix canariensis, Phoenix dactylifera, Phragmites communis, Phytolacca dioica, Pinus sylvestris, Plantago lanceolata, Platanus acerifolia, Poa pratensis, Populus deltoidesis, Quercus ilex, Quercus robur, Quercus virginiana, Robinia pseudoacacia, Rumex acetosella, Salix nigra, Salsola kali, Sambucus nigra, Schinopsis sp., Secale cereale, Taraxacum officinale, Trisetum paniceum, Triticum aestivum, Ulmus campestris, Urtica dioica, Zea mays, Canisfamiliaris, Equus caballus, Felis domesticus, Acarus siro, Blomia kulagini, Blomia tropicalis, Dermatophagoides pteronyssinus, Dermatophagoides farinae, Glycyphagus domesticus, Lepidoglyphus destructor, Tyrophagus putrescentiae, Alternaria alternata, Aspergillus fumigatus, Aspergillus niger, Cladosporium herbarum, Penicillium notatum, Rhizopus nigricans,* poissons, lait et produits laitiers, oeufs et ovo-produits, noix et fruits secs, venin de méduse, venin de serpent, venin de chenille, venin d'hyménoptère, venin de diptère, venin d'insecte de l'ordre des Siphonaptères, latex (*Hevea brasiliensis*) ou les combinaisons de ceux-ci.

5. La composition pharmaceutique destinée à être utilisée selon l'une quelconque des revendications 1 à 4, comprenant un seul allergoïde ou deux ou plusieurs allergoïdes.

6. La composition pharmaceutique destinée à être utilisée selon l'une quelconque des revendications 1 à 5, dans laquelle ledit allergoïde est lyophilisé et est dissous dans un excipient approprié pour une administration par voie intradermique.

7. La composition pharmaceutique destinée à être utilisée selon l'une quelconque des revendications 1 à 6, dans laquelle ledit excipient comprend une solution physiologique saline et/ou du mannitol.

8. La composition pharmaceutique à utiliser selon l'une quelconque des revendications 1 à 7, sous forme d'une dose unique.

9. La composition pharmaceutique destinée à être utilisée selon l'une quelconque des revendications 1 à 8, dans laquelle la teneur en allergoïde par dose unitaire de 0,1 ml est comprise entre environ 0,001 $\mu$g de protéine et environ 1000 $\mu$g de protéine par dose.

10. Kit pharmaceutique comprenant une composition pharmaceutique, la composition étant destinée à être administrée par voie intradermique, la composition comprenant au moins un allergoïde et au moins un excipient, ledit allergoïde étant un allergoïde obtenu par modification chimique dudit allergène, ledit allergoïde étant présent dans la composition à la dose maximale non réactive et ladite dose maximale non réactive correspondant à la concentration d'allergoïde qui, administrée par voie intradermique, rend le test cutané négatif tel que testé sur une population spécifiquement sensibilisée audit allergène au moyen d'une étude finale de titrage avec l'allergoïde chez une population de patients sensibilisés à l'allergène et les moyens et instructions pour administrer ladite composition pour une utilisation dans le traitement d'un sujet souffrant d'une allergie à un allergène.

11. Le kit pour une utilisation selon la revendication 10, dans lequel ledit allergoïde est lyophilisé et le kit comprend au moins un flacon contenant un excipient approprié pour l'administration de l'allergène après reconstitution par voie intradermique.

Figure 1

Figure 2

Figure 3

Figure 4

Figure 5

SDS-PAGE Native Par j vs. glutaraldehyde-polymerized Par j

| | |
|---|---|
| 1. | Low |
| 2. | Par j 001-10 |
| 3,4,5 | Par j 003p-11 |

Figure 6

IMMUNOBLOTTING Native Par j vs. glutaraldehyde-polymerized Par j

1. Low
2. Par j 001-10
3. Par j 003p-11

Figure 7

ELISA INHIBITION Native Par j vs. glutaraldehyde-polymerized
Par j

Figure 8

SDS-PAGE Native Art v vs. glutaraldehyde-polymerized Art v

Figure 9

IMMUNOBLOTTING Native Art v vs. glutaraldehyde-polymerized Art v

Figure 10

ELISA INHIBITION Native Art v vs. glutaraldehyde-polymerized
Art v

**Art v 001-10 Ref. analysis: 121123E_EL SH D: 1/1**

Art v 01-10

Art v 002p-12

$y = 32.179x + 25.669$

$R^2 = 0.9624$

$y = 12.876x + 14.57$

$R^2 = 0.9427$

Figure 11

SDS-PAGE Native Der p vs. glutaraldehyde-polymerized Der p

SDS-Page Lyo Pol Der p FP 130308A

St: Bicolor standard

1: Der p SAP 001-13

2: Lyo Pol Der p FP 130308A

Figure 12

IMMUNOBLOTTING Native Der p vs. glutaraldehyde-polymerized
Der p

## Blot Lyo Pol Der p FP130308A

St: Bicolor standard

1: Der p SAP 001-13

2: Lyo Pol Der p FP 130308A

Figure 13

ELISA INHIBITION Native Der p vs. glutaraldehyde-polymerized

Der p

| | Ag 50 | Loss of potency |
|---|---|---|
| Der p 003p-13 "T0" | 0.010 | |
| Pol Der p FP 130225 | 63.217 | 99.985% |
| Pol Der p FP 130306A | 34.153 | 99.972% |
| Pol Der p FP 130308A | 55.749 | 99.983% |

Figure 14

SDS-PAGE *Apis mellifera* venom raw material vs. glutaraldehyde-polymerized *Apis mellifera* allergen extract

75_
50_
37_
25_
20_
15_
10_

St   1   2   2   2

St: Molecular weight standard

1: *A. mellifera* raw material

2: Polymerized *A. mellifera* allergen extract

Figure 15

IMMUNOBLOTTING *Apis mellifera* venom raw material vs. glutaraldehyde-polymerized *Apis mellifera* allergen extract

250_
150_
100_
75_
50_
37_
25_
20_
15_
10_

St   1   2   2   2

St: Molecular weight standard

1: *A. mellifera* raw material

2: Polymerized *A. mellifera* allergen extract

Figure 16

44

EP 2 875 828 B1

ELISA INHIBITION *Apis mellifera* venom raw material vs.
glutaraldehyde-polymerized *Apis mellifera* allergen extract

|  | Ag 50 | Loss of potency |
|---|---|---|
| Api m RM 201237 | 0.726 |  |
| Polymerized Api m FP | 74.515 | 99.025% |

Figure 17

45

Figure 18

## REFERENCES CITED IN THE DESCRIPTION

**Patent documents cited in the description**

- GB 1282163 A

- US 4269764 A

**Non-patent literature cited in the description**

- **MALET et al.** Allergologia et Immunopathologia. Gar-si, 01 September 1994, vol. 22
- GUIA PARA LA ADMINISTRACIÓN SEGURA DE MEDICAMENTOS VIA PARENTERAL. **ERNESTO SANCHEZ GÓMEZ.** Coordinator. Juan Ramón Jiménez, May 2011
- **HANS J MAASCH ; DAVID G. MARSH.** Standardized extract modified allergens allergoids. *Clin Rev Allergy.,* 1987, vol. 5, 89-106
- **PATTERSON R.** *The Journal of Allergy and Clinical Immunology,* 1981, vol. 68 (2), 85-90
- **BOUSQUET J. et al.** *J Allergy Clin Immunol.,* 1989, vol. 84, 546-56
- **GRAMMER LC et al.** *J Allergy Clin Immunol.,* 1985, vol. 76, 397-401
- **CORRADO OJ et al.** *Allergy,* 1989, vol. 44, 108-15
- Allergen Immunotherapy: Therapeutic Vaccines for Allergic Diseases. *World Health Organization (WHO),* 1998
- **C. FAULI I TRILLO.** *Tratado de Farmacia Galénica,* 1993
- Remington's Pharmaceutical Sciences. Williams & Wilkins PA, 2000
- **WEBER, K. ; OSBORN, M.** *J. Biol. Chem.,* 1969, vol. 244, 4406-4412
- **BRADFORD, M.M.** *Anal. Biochem.,* 1976, vol. 72, 248-254
- **SIRAGANIAN RP.** An automated continuous flow system for the extraction and fluorometric analysis of histamine. *Anal Biochem,* 1974, vol. 57, 388-394
- **HANS J MAASCH ; DAVID G. MARSH.** Standardized extract modified allergens allergoids. *Clin rev allergy.,* 1987, vol. 5, 89-106
- **HANS J MAASCH ; DAVID G. MARSH.** Standardized extract modified allergens allergoids. *Clin Rev Allergy,* 1987, vol. 5, 89-106
- A review of the literature and the potential for development for use in low- and middle-income countries. *PATH report-Intradermal Delivery of Vaccines,* 27 August 2009
- *Allergy,* 1998, vol. 44 (53), 2-42
- **WEBER, K. ; OSBORN, M.** *J. Biol. Chem.,* 1969, vol. 244
- **HANS J MAASCH ; DAVID G. MARSH.** Standardized extract modified allergens allergoids. *Clin rev allergy,* 1987, vol. 5, 89-106
- **M. H. PLATZER ; C. E. H. GRATTAN ; L. K. POULSEN ; P. S. SKOV.** Validation of basophil histamine release against the autologous serum skin test and outcome of serum-induced basophil histamine release studies in a large population of chronic urticaria patients. *Allergy,* 2005, vol. 60, 1152-1156